(19) **Europäisches Patentamt** / **European Patent Office** / **Office européen des brevets**

(11) **EP 1 425 570 B1**

(12) **EUROPEAN PATENT SPECIFICATION**

(45) Date of publication and mention
of the grant of the patent:
**18.03.2009 Bulletin 2009/12**

(51) Int Cl.:
**G01N 21/64** *(2006.01)*     **G01N 33/543** *(2006.01)*

(21) Application number: **02775159.3**

(22) Date of filing: **11.09.2002**

(86) International application number:
**PCT/IE2002/000133**

(87) International publication number:
**WO 2003/023377 (20.03.2003 Gazette 2003/12)**

(54) **A LUMINESCENCE-BASED SENSOR ASSEMBLY**

SENSORANORDNUNG ZUR LUMINESZENZMESSUNG

DISPOSITIF DE DETECTION REPOSANT SUR LA LUMINESCENCE

(84) Designated Contracting States:
**AT BE BG CH CY CZ DE DK EE ES FI FR GB GR IE IT LI LU MC NL PT SE SK TR**

(30) Priority: **11.09.2001 IE 20010825**
**23.01.2002 IE 20020041**
**03.07.2002 IE 20020553**

(43) Date of publication of application:
**09.06.2004 Bulletin 2004/24**

(73) Proprietor: **Dublin City University**
**Dublin 9 (IE)**

(72) Inventors:
• **MACCRAITH, Brian**
**Portmarnock,**
**County Dublin (IE)**
• **POLERECKY, Lubos**
**Dublin City University,**
**Dublin 9 (IE)**

(74) Representative: **Gates, Marie Christina Esther et al**
**c/o Tomkins & Co.**
**5 Dartmouth Road**
**Dublin 6 (IE)**

(56) References cited:
**EP-A- 0 479 345          US-A- 4 649 280**
**US-A- 4 810 658          US-A- 5 192 510**

## Description

## Introduction

The present invention relates to a luminescence-based sensor assembly of the type comprising a superstrate; a substrate mounting an emitting spot, array of spots or a layer transmitting luminescence into the substrate; an excitation source; and a detector for measuring some of the light emitted into and transmitted out of the substrate.

Conventionally, for luminescence-based sensing, luminescence molecules forming a spot, an array of spots, or a layer, are placed on a substrate and a detector is used to detect the luminescence.

A vast number of assays carried out in biotechnology and the pharmaceutical industry use surface-bound molecules such as antibodies, which can specifically bind molecules such as antigens, from a fluid, typically a liquid flowing above. If the captured molecules contain a luminescent (which may be a fluorescent) moiety or can be labelled with one, either directly or via another molecule, they can be excited by light and subsequently the luminescence emerging from these captured molecules can provide a means for detecting the specific surface captured molecules. Within the present specification the molecules that have been captured are considered as forming a first layer, whereas other molecules which have not been bound or captured may be considered as forming a second layer. The space that extends from the surface including the luminescence label defines the first layer. It is normally the molecules forming the first layer that are of interest for detection purposes.

In many of these applications, the luminescence is excited by a so-called evanescent wave, which has the advantage of exciting the luminescence at or near the substrate interface, i.e. the first layer, rather than in the bulk superstrate above the substrate, the second layer. Essentially, it is the molecules bound, or adjacent, to the surface of the substrate which are excited and the luminescent molecules that are further away from the surface are not excited and therefore, their luminescence is not transmitted to the substrate. The evanescent wave may be used to control the distance from the surface at which the materials are excited. Essentially, this is achieved due to the localisation of the electromagnetic field of the evanescent wave in close vicinity to the interface between the superstrate and the substrate. The use of such a waveform ensures that the detected light is restricted in origin to a source close to the substrate interface, which is the preferred region of interest. As the illuminating light does not impinge on other molecules above this region there can be no excitation of those molecules and hence they will not contribute to the detected luminescence.

While evanescent wave excitation is extremely useful and effective, in certain circumstances it is not practical or convenient. Excitation by the evanescent field, which is confined to a small region above the waveguide surface and used to excite fluorescently-labelled surface attached molecules, is not particularly efficient, as only a small fraction of energy of the source of the excitation light is used for the actual excitation. This is predominantly due to the following reasons:

(i) inefficient coupling of the light generated by the excitation source into the guided mode(s), and
(ii) the fraction of the optical power contained in the evanescent field is very small in comparison to the power contained within the guiding layer.

Indeed, it would be preferable to use another, more efficient method of providing the excitation. In particular, if excitation is provided by direct illumination from within the superstrate or from below the substrate, most of the optical energy provided by the source can be used for excitation. Although this configuration improves the efficiency of excitation, it also holds some disadvantages. Namely, if a source of direct illumination is used, there may be luminescence generated in the superstrate by molecules other than those captured on the surface of the substrate, namely, by molecules in the superstrate further away from the interface of the substrate and superstrate. The latter luminescence would also be delivered to the detector causing difficulties in distinguishing between the luminescence generated at or close to the interface between the substrate and the superstrate, i.e. that emitting from the first layer and the luminescence generated further away from the interface in the superstrate itself, that originating in the second layer.

US 4, 810, 658 describes a method of optical analysis of a test sample which utilises direct illumination to excite the sample. The resultant luminescence is coupled into a waveguide where it propagates along the waveguide until it exits at a side surface thereof. It is described how by selectively positioning the detector at angles about the optical axis of the waveguide that it is possible to attribute that detected light as being due to molecules bound to the surface of the waveguide. As this arrangement relies on the detection of light which has propagated within the waveguide, the emerging signal is an integration of luminescence captured along the waveguide and is therefore not suitable for discriminating between individual sources provided on the waveguide.

A further disadvantage is that the propagation of light within a waveguide requires multiple reflections on the side walls of the waveguide which leads to inevitable losses in intensity of the signal that is eventually detected. Such losses can reduce the sensitivity of the overall apparatus.

Yet a further disadvantage is the requirement for the detection system to be placed perpendicular to an end or side surface of the waveguide which increases the overall dimensions of the test configuration, thereby making it unsuitable for certain applications.

US4810658 discloses a method of optical analysis of a test sample which comprises a sample material with light-absorbing, scattering, fluorescent, phosphorescent or luminescent properties, which sample is partly in a liquid phase and partly bound to an adjacent solid surface, to discriminate the respective parts of said sample material which are located in the liquid and bound to said solid surface: comprising the steps of providing as said solid surface a surface of a transparent solid optical waveguide, and measuring light from the sample material bound to said solid surface that has passed into and through said transparent solid optical waveguide with total internal reflections and emerged from said waveguide at an angle that deviates from the optical axis of said waveguide by an angle appreciably less than $\alpha$, where

$$\alpha = \arcsin \sqrt{(n_2^2 - n_1^2)}$$

where $n_2$ is the refractive index of the material of the waveguide and $n_1$ is the refractive index of the adjacent liquid, thus excluding from said measurement substantially all light that has emerged from said waveguide at an angle that deviates from said optical axis by alpha or more.

US5192510 discloses a system which uses the evanescent wave detection of particles to distinguish bound from free in an analyte-binding assay. Illumination below the critical angle is employed and a beveled window is used to eliminate bulk fluorescence from the emitted evanescent wave liquid. The sample is held in a non-rigid film cuvette.

There is therefore a need for a system and method that can be used for the detection of surface-generated luminescence which employs the excitation of the luminescent molecules by direct illumination, i.e., using the full power of the source of the excitation light, and yet can be used to selectively discriminate between the source of the luminescence.

In this specification, the term "critical angle" is used in its conventional sense as being Arcsin $N_2/N_1$ or Sin$^{-1}$ $N_2/N_1$ where $N_1$ is the refractive index of the optically denser material called the substrate and $N_2$ is the refractive index of the less dense material, usually called the superstrate. Generally, the superstrate is the environment in which the surface binding or luminescent material is present, typically water or air. The condition $N_2 < N_1$ must be satisfied.

The present invention is directed towards providing a means and apparatus for detecting that luminescence emitted into a substrate, which is emitted from molecules close to the superstrate/substrate interface but excited by direct illumination.

## Statement of Invention

According to the present invention, there is provided a luminescence-based sensor assembly comprising a superstrate; a substrate mounting an emitting layer capable of transmitting luminescence into the substrate; an excitation source; and a detector for measuring some of the emitted light in the substrate which is subsequently transmitted out of the substrate, characterised in that the excitation source provides direct illumination and the detected luminescence originates from a close vicinity of the superstrate/substrate interface.

Desirably, the excitation source is in the superstrate remote from the substrate. Typically the excitation source is provided above the substrate, although it will be appreciated that direct illumination may equally be effected by the provision of a source below the substrate.

The invention utilises the concept that the angular emission pattern from luminescent, typically fluorescent moieties depends strongly on proximity to the superstrate/substrate interface. Based on this, the invention applies angle-selective detection principles to discriminate between the luminescence from the surface-bound moieties and those located in the bulk of the fluid, that is to say, in the superstrate above the superstrate/substrate interface. Essentially, this is arranged by ensuring that only light transmitted into the substrate and propagating in a particular angular range above the critical angle of the superstrate/substrate interface is detected. Within the present invention the term "first layer" will be used to describe those moieties which are bound or adjacent to the substrate and whose luminescence is of interest, and the term "second layer" to all those other moieties.

Putting it in another way, a key feature of the present invention is that no light emerging from a sufficiently large distance above the substrate/superstrate interface, which emanates from inside the superstrate, can be propagated within the higher refractive index substrate at angles greater than the critical angle. However, when the source of the luminescence is close to the surface of the two materials, the radiation can be coupled into the waves propagating in the higher refractive index medium at angles greater than the critical angle. Consequently, detection of the luminescence in a particular range of angles greater than the critical angle provides the means of detecting the light originating from molecules located at or close to the surface.

One way of achieving this is by providing a light barrier in or on the substrate, which light barrier is arranged to block any light, which has been transmitted into the substrate at an angle below the critical angle.

Alternatively, the barrier can be mounted on the detector so that any light transmitted through the substrate from the emitting molecules at an angle below the critical angle, will be blocked from detection.

Another way of achieving the detection of the light radiated in the substrate at angles greater than the critical angle is to configure the substrate internally or externally so that only the light propagating at angles greater than the critical angle is redirected towards the detector.

Accordingly the invention provides a luminescent sensor configuration for use in a medium having a first refractive index, the sensor configuration comprising a source of direct illumination, a substrate having an upper and lower surface and being of a second refractive index, a material capable of luminescence, a detector arrangement provided below the lower surface of the substrate and adapted to detect light emitted through that lower surface, and wherein, in use, the medium and the substrate meet along the upper surface of the substrate which defines the boundary between the first and second refractive indices, the material capable of luminescence is excited by the source of direct illumination, thereby luminescing and the detector arrangement is adapted to discriminate between luminescent light emitted from a region within a predetermined distance of the upper surface and light emitted from any other regions, the discrimination being effected by selective detection of light emitted from the luminescent material at angles greater than the critical angle of the medium/substrate interface.

The angles greater than the critical angle are desirably angles within a specific range which are predetermined for optimum performance of the system.

Desirably, the predefined distance is within the range of upto about 4 $\lambda$, desirably about 0.5 $\lambda$ to about 3 $\lambda$, and more preferably within the range of about 1 to about 2 $\lambda$, wherein $\lambda$ is the wavelength of the luminescence light.

As detailed above, the luminescent molecules contained within the predefined distance may be considered as forming a first layer whereas those molecules or materials outside that distance may be considered as forming a second layer.

The angle at which the luminescence is emitted into the substrate and subsequently selectively detected is preferably further greater than a threshold angle, the threshold angle being an angle which satisfies the equation:

$$I_s(\theta_{tr}) / I_b(\theta_{tr}) = F_{tr},$$

where Is $(\theta_{tr})$ is the intensity of light emitted from the first layer at the threshold angle, $I_b(\theta_{tr})$ is the intensity of light emitted by the second layer at the threshold angle and $F_{tr}$ is a confidence or performance factor which is selected by the user. $I_b(\theta_{tr})$ typically corresponds to a background level within the configuration system such that the equation reduces to providing a threshold angle which satisfies the equation that the signal-to-background ratio of the measurement of the luminescence originating from the first layer is greater than some specified value $F_{tr}$.

The background level may in certain circumstances be considered a noise level for the system, although it will be appreciated that there are many different contributions within a system that may affect the overall background level.

In a first embodiment the first and second layers have the same refractive index. In an alternative embodiment, the first and second layers have a different refractive index.

In one embodiment, the light may be emitted into the substrate from more than one source and the detector arrangement is adapted to spatially discriminate between the origins or sources of the detected light.

Typically, the configuration includes at least one portion of capture material adapted to capture a specific target species, the at least one portion of capture material being coupled to the substrate and adapted, in use, to capture any of a predefined target substance within the medium, the capture or tagging effecting the formation of a captured species, which either directly or indirectly is adapted to luminescence upon excitation, such luminescence being detectable by the detector.

The captured species or material may be directly capable of luminescence or may require a subsequent combination with a further material to effect the formation of a luminescent source, thereby forming an indirect source of luminescence.

In certain embodiments at least two distinct portions of capture material are provided, each portion being coupled to the substrate and wherein the substrate is configured to redirect light emitted by each portion towards the detector such that the light received at the detector from a first portion is spatially independent from the light received at the detector from a second portion.

The light detected by the detector may be detected without undergoing total internal reflection within the substrate prior to detection.

Desirably, the detector arrangement includes at least one optical redirection element at either an upper or lower surfaces of the substrate, the optical redirection element adapted to redirect light emitted by the luminescent material into the substrate at an angle greater than the critical angle out of the substrate and towards a detector.

This at least one optical redirection element may be adapted to redirect the light using total internal reflection.

A plurality of optical redirection elements may be provided, each element comprising a frusto-conical structure raised above the upper surface of the substrate, each frusto-conical structure having side walls and an upper surface, luminescent material being carried on the upper surface of the structure, and wherein light emitted by the material into the structure is internally reflected by the side walls of the structure and directed towards a detector positioned beneath the substrate.

Alternatively, a plurality of optical redirection elements, each element in the form of a ridge raised above the upper surface of the substrate and extending along the upper surface of the substrate may be provided, the ridge having side walls and an upper surface, luminescent material being carried on the upper surface of the ridge, and wherein light emitted by the material into the ridge is internally reflected by the side walls of the ridge and directed towards a detector positioned beneath the substrate.

The at least one optical redirection element may be adapted to redirect the light using refraction. Such an element may be in the form of one or more prisms optically coupled, or integral, to a lower surface of the substrate, the prism being adapted to receive light incident on the lower surface of the substrate and redirect that light sidewardly towards a detector.

In another embodiment the at least one optical redirection element is adapted to redirect the light using diffraction, which may be provided by a diffractive optical element provided at the lower surface of the substrate.

The lower surface of the substrate may be structurally configured to both reflect and refract light radiated into the substrate, the reflection and refraction of the light effecting a redirection of light towards a detector, the light redirected being that light having propagating within the substrate at an angle greater than the critical angle of the substrate/medium interface.

The selective detection of light may be effected by providing the substrate with non-parallel upper and lower surfaces, the angle of the upper and lower surfaces being such that the light emitted by the luminescence material is incident on the surfaces at angles greater than the critical angle of the substrate/medium interface, thereby effecting a propagation of light along an axis of the substrate towards a detector.

The sensor configuration may be further modified so as to detect light radiated into the substrate by the luminescent material at angles which are not less than the critical angle of the luminescent material/substrate interface and greater than the critical angle of the medium/substrate interface.

The detector is desirably a CMOS, a CCD or a photodiode type detector, which can be located at a specific location below the substrate.

The sensor may be provided initially with a bio-recognition element, the bio-recognition element being sensitive to and adapted to couple with a compatible biological sample of preselected variety in the medium with which the sensor is used. In such element types, a combination of the bio-recognition element with the preselected sample variety effects the formation of a material capable of luminescence. In other element types, a sandwich assay is formed by a further coupling of the coupled biological sample/bio-recognition element with a luminescent tag or label to effect the formation of the luminescent material.

The invention arises out of our analysis of the radiation of dipoles placed above a higher refractive index substrate which reveals that the luminescence exhibits strong spatial anisotropy, with significantly greater amounts of luminescence radiated within a certain interval of angles. It has been appreciated by the present inventors that a significant amount of luminescence is radiated into the higher refractive index substrate at angles greater than the critical angle of the substrate/superstrate interface. Thus, in most substrates, a significant amount of the luminescence is radiated into the substrate and is trapped there. Accordingly, the idea is to provide a range of configurations which exploit these findings and ensures that the luminescence, instead of being trapped permanently within the substrate, is transmitted out of it for subsequent detection and measurement.

Our analysis of the radiation of dipoles placed above a higher refractive index substrate also reveals that the luminescence originating from molecules which are located further away from the substrate/superstrate interface than some specific distance, denoted by $t_s$, cannot propagate within the substrate at angles greater than some specific angle, denoted by $\theta_s$. However, the luminescence originating from molecules which are located within the distance $t_s$ above the substrate/superstrate interface can emit light which is propagating in the substrate at angles greater than $\theta_s$.

In one embodiment of the invention, the luminescence-based sensor is so arranged that the light is directed through the exit surface substantially normally thereto.

In another embodiment of the invention, at least either the upper surface mounting the emitter or the lower surface of the substrate is not planar. If planar, the surfaces are not parallel.

In one embodiment of the invention, the interfaces of the substrate are so configured that the internal reflection at the interface on which the light impinges is substantially prevented and allows the light to be transmitted through the substrate.

In another embodiment of the invention, the interfaces of the substrate are so configured that the light is reflected from at least one interface before being directed out of the substrate to the detector.

## Brief Description of the Drawings

The invention will be more clearly understood from the following description of some embodiments thereof, given by way of example only, with reference to the accompanying drawings, in which: -

Figure 1 shows the angular properties of luminescence radiated from a small luminescence spot located on a glass substrate; the substrate being surrounded by air below and by air above ( Figure 1a) and by water above (Figure 1b),
Figures 2(A) and (b) are side views showing the effect of light generated at different distances from the surface of a substrate.

Figures. 3a and 3b are side views of luminescence-based sensor assemblies.

Figure 4 shows the angular distributions of intensity of luminescence radiated by a dipole located in air (a) and water (b) at various distances $t_d$ from the glass substrate.

Figure 5 is a schematic diagram of a thin dipole layer (refractive index $n_1 = 1.43$, thickness $t_1$) deposited on a planar glass substrate, with the environment covering the layer being either air or water.

Figure 6 shows angular distributions of intensity of the luminescence radiated from the configuration of Figure 5, with the environment covering the layer being air (a) and water (b).

Figure 7 is a schematic diagram of a two-layer system comprising a thin luminescent layer (refractive index $n_1 = 1.43$, thickness $t_1$) and a thin buffer layer (refractive index $n_1 = 1.43$, thickness $t_b$) deposited on a planar glass substrate.

Figure 8 shows the angular distributions of intensity of the luminescence radiated into the glass substrate and originating from the two layer system of Figure 7, with the system being covered by air (a) and water (b).

Figure 9 shows schematic diagrams of two-layer systems consisting of a glass substrate, a sol-gel layer and a bulk layer, the structures being covered by water, with (a), (b) and (c) corresponding to the following situations: (a) the bulk layer contains luminescent molecules while the sol-gel layer does not; (b) the bulk layer does not contain luminescent molecules while the sol-gel layer does; (c) both the bulk and sol-gel layers contain luminescent molecules.

Figure 10 shows the angular distributions of intensity of luminescence generated by a multilayer structure shown in Figure 9, the graphs (a) and (b) correspond to the situations depicted in Figs. 9(a) and 9(b), respectively with the different lines in Figure 10 (a) corresponding to different values of the thickness of the bulk layer tb in Figure 9(a), as indicated by the legend of the graph in Figure 10(a).

Figure 11 is an example of the angular distribution of intensity generated by a multilayer structure shown in Figure 9(c), with different lines corresponding to different values of the thickness of the bulk layer tb in Figure 9(c),as indicated by the legend of the graph.

Figure 12 is a schematic diagrams of a two-layer system consisting of a glass substrate covered with water, with (a), (b) and (c) corresponding to the following situations: (a) the bulk layer contains luminescent molecules while the surface layer does not; (b) the bulk layer does not contain luminescent molecules while the surface layer does; (c) both the bulk and surface layers contain luminescent molecules.

Figure 13(a) shows angular distributions of the total intensity of luminescence radiated into the glass substrate, which is given as a sum of the contributions originating from the "surface" and "bulk" layers, with different lines corresponding to different values of the thickness of the bulk layer in Figure 12(c), as indicated by the legend.

Figure 13(b) shows the angular distribution indicating separate contributions to intensity of the luminescence originating from the surface layer of thickness ts (solid line) and the bulk layer of thickness tb (dash-dotted line).

Figure 14(a) shows angular distributions of the luminescence radiated by a 2-layer system depicted in Figure 12(c) with the curves denoted by (BL) and (SL) corresponding to the situations where the luminescence originates from the bulk and surface layers, respectively.

Figure 14(b) shows the threshold angle as a function of the surface layer thickness for two different values of the threshold factor $F_{tr}$.

Figure 15a shows a sensor arrangement comprising a prism.

Figures 15b to 15d show modified structures for detecting luminescence according to embodiments of the present invention.

Figure 16 shows an exemplary array structure.

Figure 17 shows an alternative array structure.

## Detailed Description of the Drawings

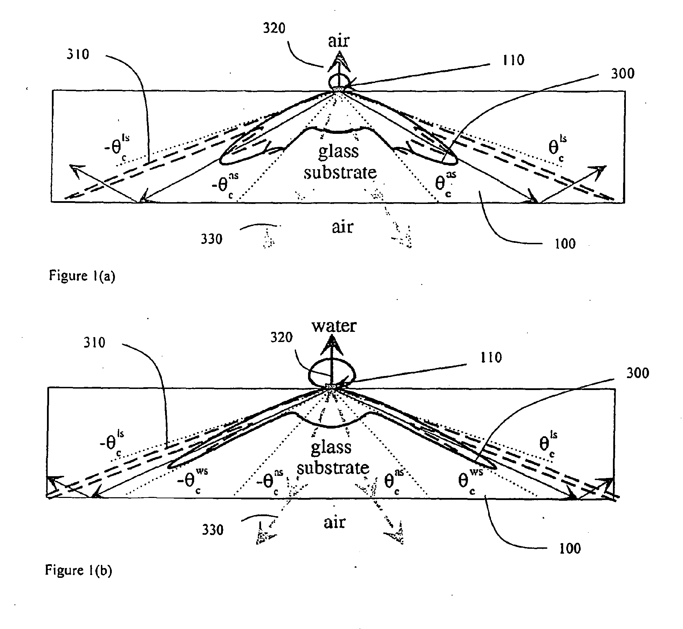

Figures 1 shows an example of a sensing element. The same reference numerals will be used for the same components in the various embodiments. It consists of a "thick" glass slide substrate 100 (refractive index $n_s = 1.515$, thickness - 1mm) on top of which a small spot of luminescent material 110(refractive index $n_1 = 1.43$) is deposited. It will be appreciated that the material is optically coupled to the substrate. By the term optically coupled it will be appreciated by those skilled in the art that it encompasses a plurality of different arrangements including, but not limited to:

    i. luminescent molecules directly bound to or adsorbed on a substrate,

    ii. luminescent molecules indirectly attached to substrate via one or more linker molecules (such as in a sandwich assay),

    iii. luminescent molecules entrapped/contained within a thin film, for example a polymer or sol-gel matrix, coated on substrate.

    iv. a layer of living cells containing luminescent centres, such as auto fluorescent bacteria.

The thickness $t_1$ of the layer forming the spot is assumed to be uniform and typically in the range of hundreds of nanometres. Typically, the dimensions of such a spot are determined by the application and will be defined by the user. Furthermore, for simplicity, the size of the spot is assumed to be small compared to the size of the area of the detection system, which is used to detect the luminescence produced by the spot. The latter restriction is assumed only to ensure that the luminescent spot "appears" to the detector as a spot rather than as an area over which the radiated intensity would have to be integrated. Consequently, the lateral (x-y) dimensions do not have to be considered and only the angular dependence of the radiated intensity needs to be taken into account in the following analysis. The luminescent spot is assumed to be covered by the environment, which is either air ($n_a$ = 1.0) or water ($n_w$ = 1.33). The slide is surrounded by air from below.

The predicted angular distribution of the luminescence emerging from the small luminescent spot deposited on the glass substrate is shown in Figure 1. The graphs (a) and (b) correspond to the situations where the environments or media covering the spot are air and water, respectively. In both graphs, the solid line 300 and the dashed line 310 correspond to the thickness of the luminescent spot equal to $t_1 = 0.5\lambda$ and $t_1 = 1.5\lambda$, respectively, where $\lambda$ is the luminescence wavelength. Luminescence that can be detected by the detector placed above the glass substrate is schematically shown by the arrow 320. Luminescence within this angular distribution is typical of the luminescence that has traditionally been used within sensor systems. As can be seen from the displacement of the luminescence as shown in the solid 300 or dashed 310 lines located in air or water above the glass substrate, the amount of luminescence radiated into the environment covering the spot is relatively small.

The situation is similar when the detector is placed below the glass substrate. Due to reflections taking place at the bottom glass/air interface, the light impinging at this interface is transmitted to air only if the incident angle lies within the angular range $\theta \in \langle -\theta_c^{as}, \theta_c^{as} \rangle$, where

$$\theta_c^{as} = \arcsin(n_a / n_s) \approx 413^0$$ is the critical angle of the substrate (glass)/air interface. This light is schematically depicted by the dashed arrows 330. Due to the refraction, the light propagating inside the substrate at angles $\theta \in \langle -\theta_c^{as}, \theta_c^{as} \rangle$ is partially transmitted into the air under the substrate at angles $\theta \in \langle -90°, 90° \rangle$. The solid 300 and dashed 310 lines within the angular range $\theta \in \langle -\theta_c^{as}, \theta_c^{as} \rangle$ demonstrate that the amount of luminescence transmitted to air below the glass substrate is also relatively small.

The light propagating inside the substrate at angles greater than the critical angle $\theta_c^{as}$ is totally internally reflected at the lower substrate/air interface. If the environment covering the slide is air, as shown in Fig. 1(a), this light is also totally internally reflected at the upper layer/air interface and is effectively trapped (or confined) within the waveguiding glass substrate. If the environment above the slide is water, as shown in Fig. 1(b), the part of the light propagating in the substrate at angles $\theta \in \langle \theta_c^{as}, \theta_c^{ws} \rangle$ and $\theta \in \langle -\theta_c^{as}, -\theta_c^{ws} \rangle$ is partially transmitted into water and partially reflected back to the substrate. Furthermore, the part of light propagating at $\theta \in \langle \theta_c^{ws}, 90^0 \rangle$ and $\theta \in \langle -\theta_c^{ws}, -90^0 \rangle$ is totally reflected at the upper layer/water interface. In any case, due to the relation $\theta_c^{ws} > \theta_c^{as}$, the light exhibiting the enhanced intensity is always trapped inside the substrate due to the total internal reflection at both the upper and lower interfaces. For the ease of explanation the term , $\theta_c^{es}$ , can be taken as being equivalent either to $\theta_c^{as}$ or $\theta_c^{ws}$ depending on whether the environment covering the luminescent spot is air or water.

The above analysis of the radiation properties of light propagating indicates that the propagation of the light within the substrate is independent of the way the radiation was excited. It will be understood therefore that any type of excitation, which would provide the same spatial distribution of the radiating molecules, would result in the same characteristics of the radiated luminescence.

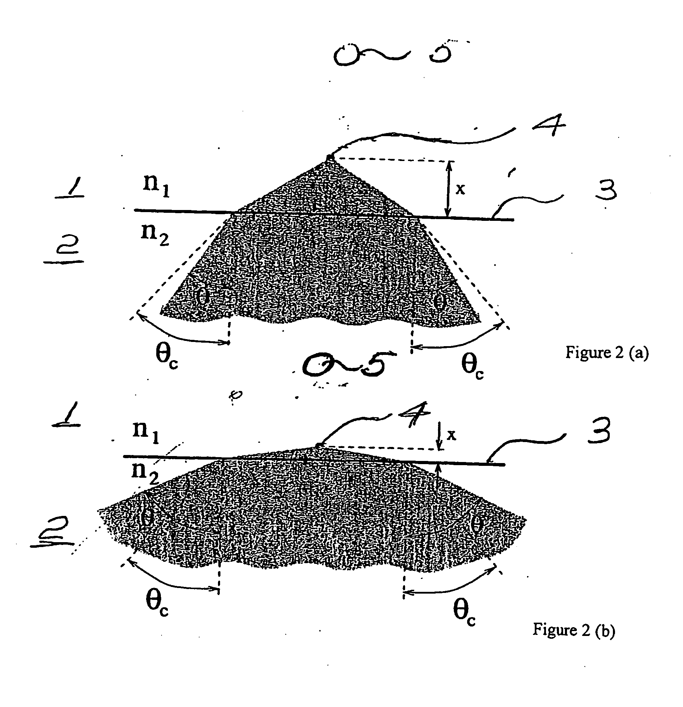

Referring to Figure. 2, there is illustrated a superstrate 1 above a substrate 2 having a surface 3 forming a superstrate/substrate interface and a luminescent source 4, for example, any form of luminescent molecule. An excitation source, namely, a light source 5 is mounted above the surface 3 and spaced-apart therefrom. The luminescence source 4 is illustrated in Fig. 2(a) and (b) at different distances X from the surface 3. Further, the superstrate 1 has a lower refractive index $n_1$ than the substrate 2 which has a refractive index $n_2$, i.e. $n_1 < n_2$.

Referring now to Fig. 2(a), when the light source 5 causes the luminescent source 4 to emit light, it will be noted that the light is emitted at a distance X, considerably greater than $\lambda$, which is the wavelength of the light being emitted. That light from the luminescence source 4 does not propagate into the substrate 2 at angles greater that the critical angle $\theta_c$. In practice, the fraction of Luminescence light in the higher luminescence refractive index substrate at angles greater than the critical angle $\theta_c$ decreases rapidly as distance X increases. Therefore, if X is chosen to be sufficiently large, for example, $X > 2\lambda$, the amount of light propagating in the substrate at angles greater than the critical angle is negligible. However, referring to Fig. 2 (b), if the luminescent source 4 is at a small distance from the superstrate/substrate interface, for example, $X < \lambda$, then a significant fraction of the luminescence light will propagate in the substrate in the range of angles above the critical angle $\theta_c$.

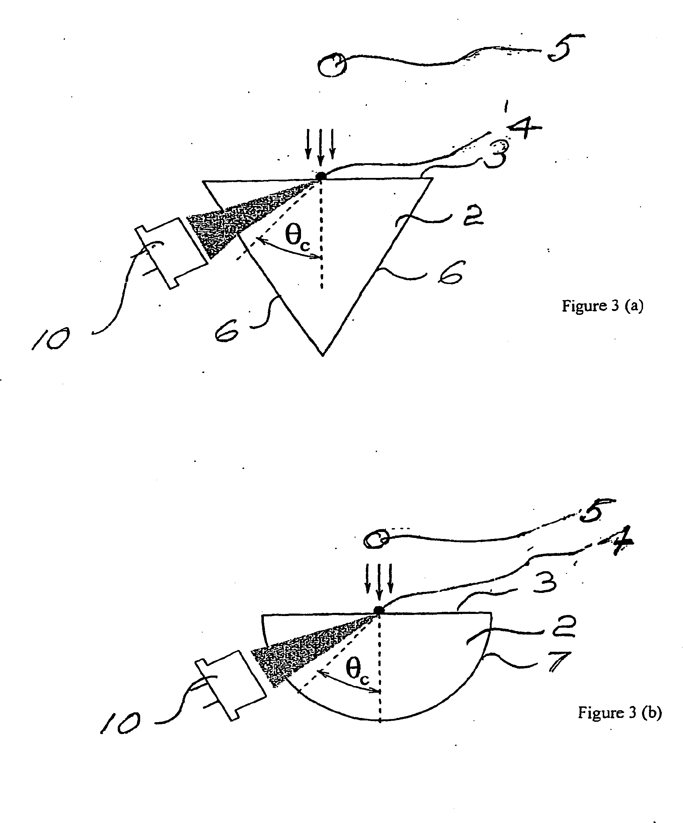

Referring to Figures 3a and 3b, with parts similar to those described with reference to the previous drawings being identified by the same reference numerals, two embodiments of the present invention are illustrated. In these embodiments, the drawings are identified by the same reference numerals. In the embodiment of Figure 3a, the excitation source 5 is again placed above the substrate 2 which is in the form of a prism. A luminescent source 4 is attached to the surface 3. A photodetector, in this embodiment, a CCD camera 10, is mounted adjacent one of the planar surfaces 6 of the substrate 2 for capture of light propagated in a particular range of angles above the critical angle $\theta_c$. In the embodiment of Figure 3b the substrate, again identified by the reference numeral 2, is again in the form of a prism having an arcuate lower surface 7. The prism is adapted to direct light propagating within a range of angles above the critical angle from the luminescence source 4 onto the detector 10. Due to the configuration of the prism surface the light that is propagating within the substrate, although it may be at angles greater than $\theta_c$ is incident on the surface of the prism at angles less than the critical angle and is therefore able to out-couple from the substrate and may be detected.

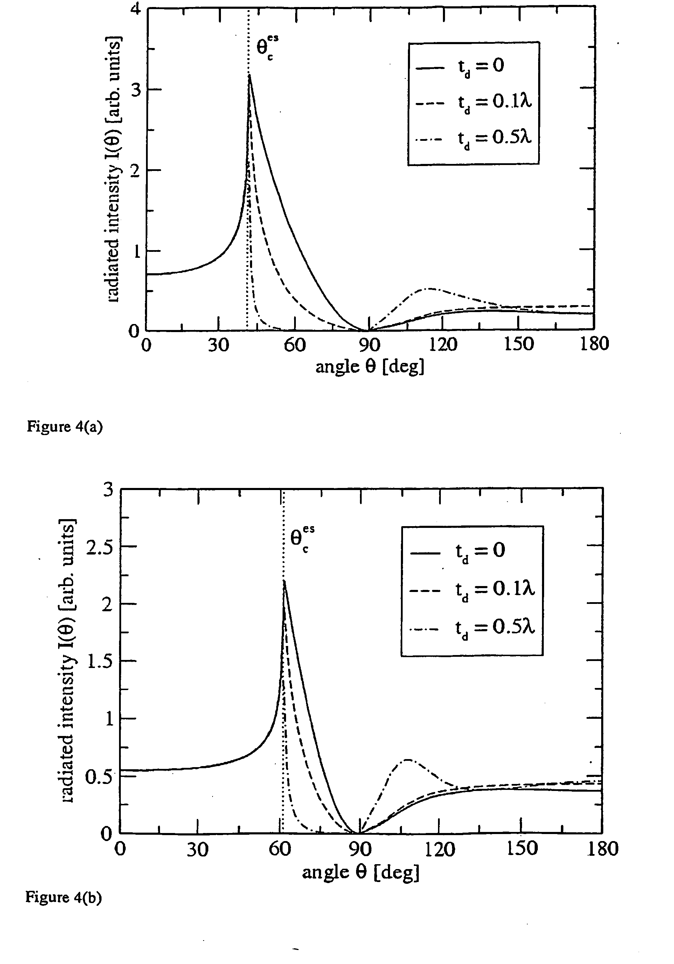

If one assumes the luminescent source to behave as a radiating dipole such as what is described in Polerecky et al ( Applied Optics 39 (22): 3968-3977 Aug 1 2000), it can be shown that the angular distribution of the intensity of the light radiated below the critical angle does not appreciably change with the distance of the dipole from the substrate. Figures 4a and 4b, which correspond to the situation where the environment covering the substrate is air and water respectively, show the angular distributions of intensity of the radiated luminescence for three distances of the radiating dipole from the glass substrate-corresponding to distances equivalent to 0, 0.1$\lambda$ and 0.5$\lambda$.

As can be seen from the graphs in Fig. 4, the angular distribution of the intensity radiated below the critical angle $\theta^{es}_c$ does not change with the distance $t_d$ of the dipole from the glass substrate. The intensity radiated into the environment, i.e., at angles $\theta \in \langle 90°, 180° \rangle$ varies in that its total amount increases with increasing value of $t_d$.

Furthermore, a peak starts emerging at $\theta \approx 100°$ for greater values of $t_d$. Although the present invention is not intended to be limited to any one specific theory it is thought that this is due to interference of the luminescence radiated directly into the environment and that reflected from the environment/substrate interface. The number of these peaks, which form a fringe-like pattern in the angular distribution of the intensity, would increase with increasing distance $t_d$ (not shown in the Figure).

The most significant changes in the intensity profile are observed within the angular range

$$\theta \in \left\langle \theta_c^{es}, 90^0 \right\rangle, \text{ where } \theta_c^{es} \text{ is either}$$

$$\theta_c^{as} = 41.3^0 \text{ or } \theta_c^{ws} = 61.3^0,$$ depending on

whether the environment is air or water, respectively. In particular, the intensity fall-off above the critical angle is more abrupt for greater distances $t_d$. Furthermore, for a distance as low as $t_d = 0.5\lambda$, there is almost no luminescence radiated above the critical angle $\theta_c^{es}$ , as shown by the dash-dotted line. These important features can be explained as follows. The electromagnetic field, which propagates in the glass substrate at angles $\theta \in \left\langle \theta_c^{es}, 90^0 \right\rangle$ is exponentially decreasing in the environment. A characteristic penetration depth of this so-called evanescent field is approximately $\lambda$ and it decreases with the increasing propagation angle $\theta$. Because the luminescence at these angles is provided by coupling of the dipole's near-field with the evanescent field, it is understandable that its intensity is decreasing for increasing $\theta$. Moreover, for a sufficiently large distance of the dipole from the surface, the evanescent field does not reach the dipole's position. This implies that there is very little luminescence radiated above the critical angle for such large distances due to a weak coupling of the evanescent field and the dipole's near-field, as concluded above.

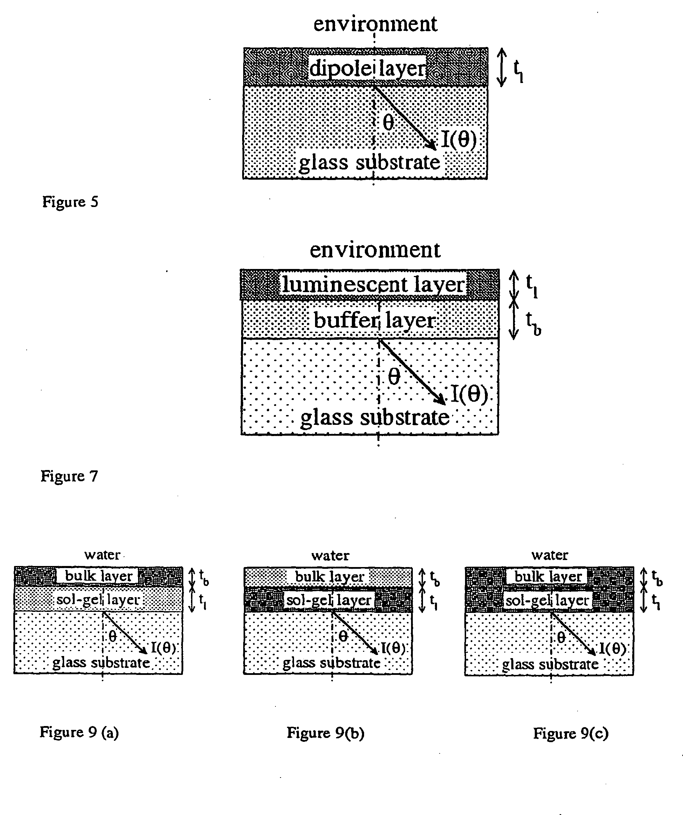

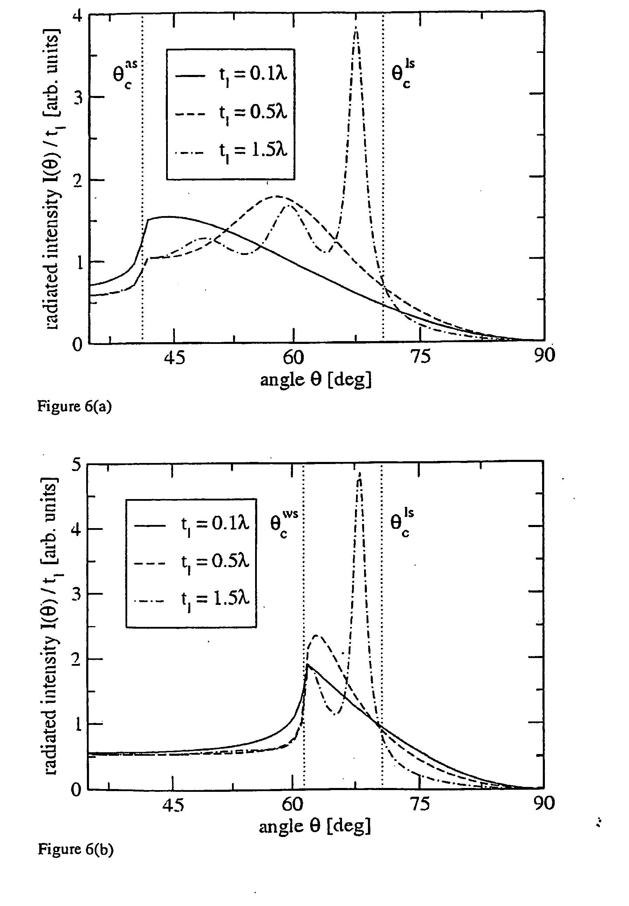

The above description was with reference to a single radiating dipole provided at various distances above a substrate. Figures 5 and 6 show an analysis of varying the thickness of a thin dipole layer deposited on a planar glass substrate, with the environment being either air (Fig. 6a) or water (Fig. 6b). Figure 6 shows the angular distributions of intensity of the luminescence radiated from the luminescent layer whose thickness takes values $t_1 = 0.1\lambda$, $0.5\lambda$, and $1.5\lambda$, where $\lambda$ is the wavelength of luminescence. Only the intensity radiated into the glass substrate is shown. As can be seen, the angular distribution at angles below the critical angle $\theta_c^{es}$ does not change significantly with the thickness of the dipole layer, and is close to that corresponding to the point dipole radiation described above with reference to Figure 4.

Notable changes are observed at angles $\theta \in \left\langle \theta_c^{es}, \theta_c^{ls} \right\rangle$; where $\theta_c^{ls} = \arcsin(n_1/n_s) \approx 70.7°$ is the critical angle of the layer/substrate interface. Within this angular range, the angular distribution of intensity exhibits a distinct peak. This peak is more pronounced and shifted towards $\theta_c^{ls}$ for greater values of the thickness of the dipole layer. Furthermore, at these greater thickness', the sharp peak is accompanied by several less significant peaks, which "emerge" from the angular position determined by $\theta_c^{es}$ . This is demonstrated by the dash- dotted line in Fig. 6(a). This feature is not yet visible in Fig. 6(b) as the thickness of the luminescent layer is not sufficiently large.

The behaviour of the radiated intensity described above can be qualitatively understood by considering the following arguments. The electromagnetic field, which corresponds to the modes propagating in the glass substrate at angles $\theta \in \left\langle \theta_c^{es}, \theta_c^{ls} \right\rangle$, is propagating within the dipole layer. Due to interference effects caused by the reflections at the substrate/layer and layer/environment interfaces, the magnitude of the field can be considerably enhanced for a certain value of the angle θ. The coupling efficiency between the near-field of the dipoles inside the layer and the far-field propagating in the glass is proportional to the magnitude of the field inside the dipole layer. Therefore, the enhancement of the radiated intensity at a particular angle θ is a consequence of the enhancement of the field corresponding to the modes propagating at this angle.

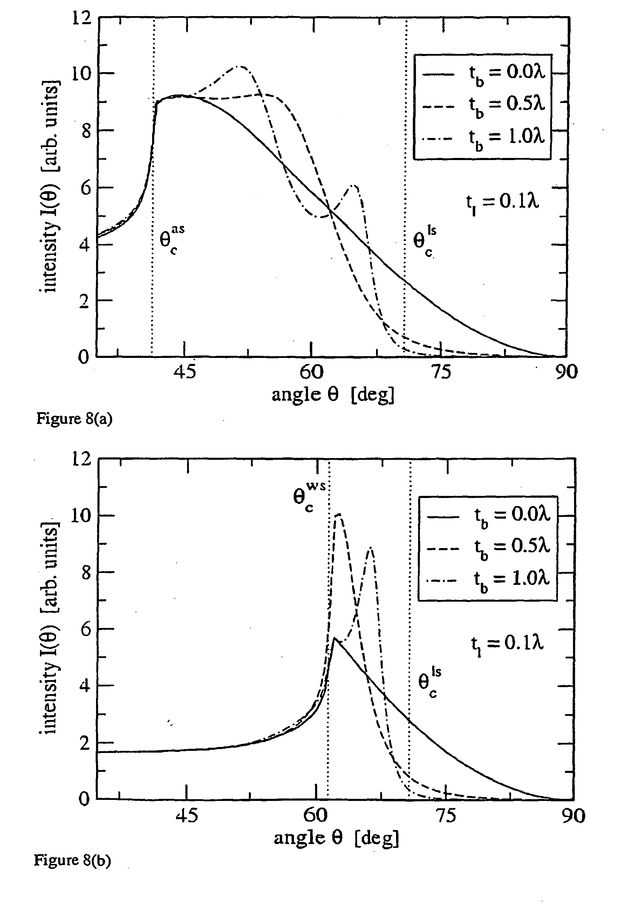

Figures 7 and 8 show an extension of this analysis to a two-layer system comprising a glass substrate covered by a buffer layer of refractive index $n_1$=1.43 and variable thickness $t_b$. On top of the buffer layer is provided a luminescent dipole layer of refractive index $n_1$=1.43 and thickness $t_1$=0.1λ. The luminescent layer is covered by an environment, which is either water or air. This specific example illustrates the influence of a buffer layer thickness on the angular profile of intensity of the radiated luminescence.

The angular dependence of intensity of the luminescence radiated into the glass substrate is shown in Fig. 8. The graphs (a) and (b) correspond to the situations where the luminescent layer is covered by air and water, respectively. The thickness of the buffer layer varies between $t_b$ =0λ; 0.5λ, λ. As can be seen, the influence of the buffer layer on the angular profile of the radiated intensity is twofold. Firstly, in the angular range

$$\text{range } \theta \in \left\langle \theta_c^{es}, \theta_c^{ls} \right\rangle,$$ the smooth decrease of the intensity with the increasing angle θ is changed to a more complex profile containing peaks and dips, the number of which depends on the thickness $t_b$ of the buffer layer. These peaks are due to the same interference effects as those discussed above with reference to a layer deposited directly on the substrate. The second important influence of the buffer layer can be observed at angles above the critical angle $\theta_c^{ls}$ of the buffer layer/substrate interface. The total amount of luminescence radiated above this angle is decreased substantially even for as thin a buffer layer as $t_b$ = λ (see the dash-dotted line). This is due to the same reasons as already discussed above.

In particular, the field corresponding to the intensity observed at these angles is evanescent in the buffer layer. When the thickness of the buffer layer is sufficiently large, the field barely reaches the luminescent layer, which decreases the coupling efficiency between the near-field of the radiating dipoles and the radiated field. Consequently, the amount of luminescence propagating in the glass substrate at angles $\theta > \theta_c^{ls}$ is

very small for greater values of $t_b$.

Figure 9 illustrates an exemplary configuration similar to that discussed in the previous section. The results obtained from this numerical analysis are particularly applicable to practical applications where the surface-generated luminescence is of interest. The two-layer system consists of a glass substrate, which is covered by a sol-gel layer of refractive index $n_1$ = 1.43 and thickness $t_1$ = 1.5λ. It will be appreciated that the values presented here are exemplary of the values that may be used in configurations and it is not intended to limit the present application to any particular value of $n_1$ or $t_1$; $n_1$ simply has to be smaller than the refractive index of the substrate and greater than the refractive index of the superstrate, and that the values give here are illustrative of typical values. This layer is either luminescent or non-luminescent. On top of this layer is a bulk layer of water (thickness $t_b$), which either does or does not contain luminescent molecules. The purpose of this bulk layer is to model the contribution to the radiated luminescence originating from the volume above the thin sol-gel layer. The bulk layer is covered by water free of luminescent molecules.

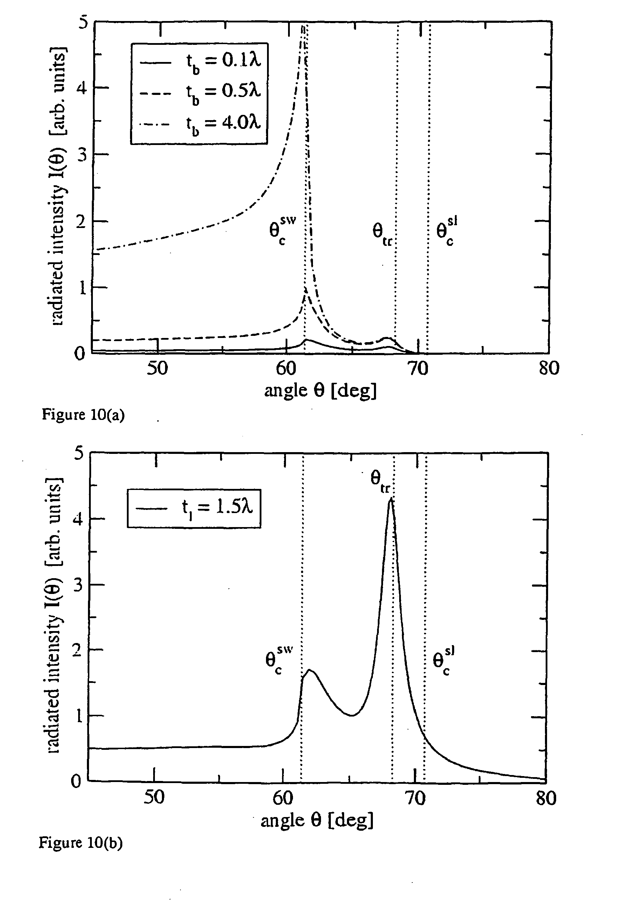

Firstly, it is considered that the sol-gel layer does not and the bulk layer does contain luminescent molecules, as shown in Fig. 9(a). The corresponding angular profile of intensity of the luminescence radiated into the glass substrate is shown in Fig. 10(a). As can be seen, the luminescence radiated from the bulk layer can be observed mainly at angles below the critical angle of the water/substrate interface $( \theta_c^{ws} )$. The greater is

the thickness $t_b$ of the bulk layer, the greater is the amount of luminescence observed below the critical angle $\theta_c^{ws}$.

On the other hand, the contribution of the bulk layer to the luminescence observed within the angular

range $\theta \in \left\langle \theta_c^{ws}, \theta_c^{ls} \right\rangle$ is small and does not significantly change when the bulk layer thickness exceeds the value of approximately 4λ, as demonstrated by the dash and dash-dotted lines in Fig.10(a). This is an important observation because it enables one to extend the thickness of the bulk layer to an arbitrarily large value without modifying the angular distribution of the luminescence radiated within this angular range. It should also be noted that there is only a negligible contribution of the bulk layer to the luminescence observed above the critical angle

$\theta_c^{ls}$.

If one considers the opposite case, i.e. where the sol layer does and the bulk layer does not contain luminescent molecules, which is shown in the example of Figure 10b, then it can be shown that the main contribution to the luminescence originating from the sol-gel layer is observed at angles $\theta \in \left\langle \theta_c^{ws}, \theta_c^{ls} \right\rangle$. Further-

more there is a considerable amount of luminescence radiated above the critical angle $\theta_c^{ls}$.

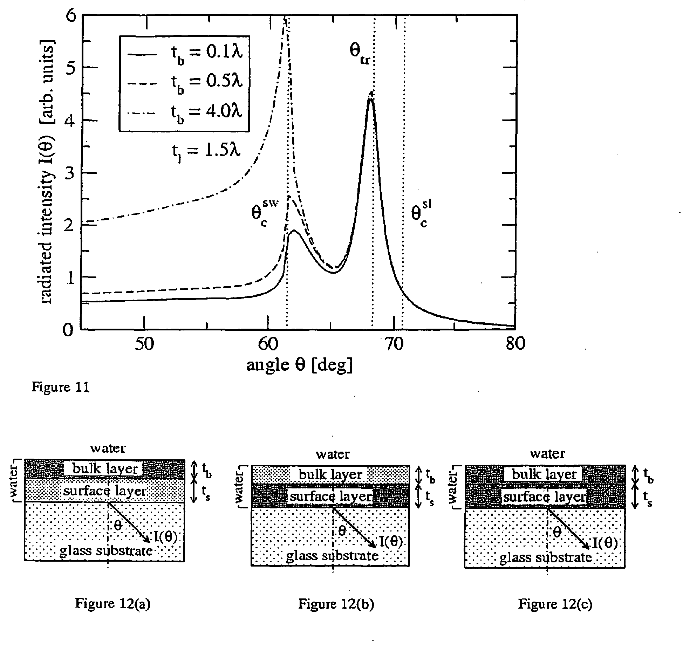

In the scenario where both the sol gel layer and the bulk layer contain luminescent material and assuming that both the layers have equal densities of molecules, as is shown in Figure 9(c), then the angular distribution of intensity resembles that shown in Figure 11. Due to the fact that the contributions to the luminescence originating from different parts of the structure are considered to be uncorrelated (in the statistical sense), the total intensity is given by the sum of the contributions from the sol-gel and bulk layers. It will thus be appreciated that the graph demonstrates that it is possible to distinguish between the contributions originating from the doped sol-gel layer and the luminescent bulk layer. This is due to the fact that these two contributions are observed within different angular regions. In particular, the main contribution originating from the bulk layer is radiated at angles below the critical angle $\theta_c^{ws}$, while the main contribution originating from the thin sol-gel layer is observed at angles above the critical angle $\theta_c^{ws}$. Although the distinction between the two contributions is not sharp around the critical angle $\theta_c^{ws}$, there is a definite angle, denoted by $\theta_{tr}$ above which the contribution originating from the bulk layer is negligible in comparison to the contribution originating from the sol-gel layer. The value of this angle can be determined by combining this analysis and the background signal characteristics of the particular detection system. It will therefore be appreciated that by applying the technique of the present invention that it is possible to discriminate in the light detected at a detector where that light originated, i.e. whether it is due to luminescence of luminescent molecules in a region close to the substrate interface or whether it is due to the luminescence of the molecules outside that region.

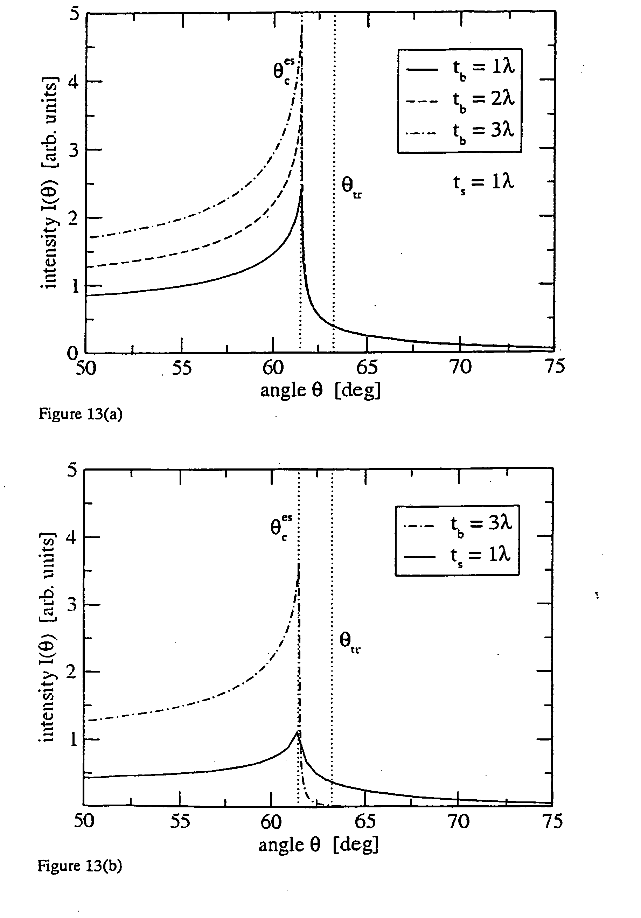

Figure 12 shows the application of the analysis relating to the dipole activity between a surface and bulk contribution so as to provide for a technique suitable for distinguishing between the surface and bulk-generated luminescence. Such application has particular importance in sensor applications which are used in an in situ environment where the sample being tested is flowing through the cell and the user wishes to discriminate in real time between the luminescence originating from the molecules located near the substrate /environment interface and that originating from the molecules still flowing through the cell. The structure under consideration consists of a glass substrate covered by water. The water environment is formally divided into a "surface" layer, a "bulk" layer and the bulk itself. This formal division has been introduced to enable the modelling of the properties of the luminescence originating from the bulk located above the surface layer, and is representative of a region within a predetermined distance of the interface on the medium side of the interface and a second region outside that predetermined distance. The surface layer is a layer of water of thickness $t_s$ adjacent to the glass substrate. The bulk layer is another layer of water (thickness $t_b$) covering the surface layer. The purpose of this division is to model the contributions to the radiated luminescence originating from molecules located close to the surface of the substrate and those located further away from the surface. The situation is depicted in Fig. 12. Firstly, it is assumed that both the surface and bulk layers contain luminescent molecules. This means that the luminescence originates from a layer of thickness $t_s + t_b$, as shown in Fig. 12(c). The angular distribution of intensity of the luminescence radiated into the glass substrate from such a system is shown in Fig. 13 (a) for $t_s = \lambda$ and various values of the bulk layer thickness (see the legend of the graph). As can be seen, the increased value of the bulk layer thickness results only in increase of the level of luminescence intensity essentially below the critical angle of the environment/substrate interface $\theta_c^{es}$. Above this angle, the intensity of luminescence remains practically unchanged for all values of the bulk layer thickness $t_b$.

To explain this behaviour, the contributions originating from the surface and bulk layers are plotted separately.

This is shown in Fig. 13(b) by the solid and dash-dotted lines, respectively. The dash-dotted line indicates that the luminescence originating from the bulk layer falls rapidly above the critical angle $\theta_c^{es}$. Above the threshold value of the observation angle $\theta_{tr}$, the luminescence intensity arising from the bulk is negligible in comparison with the contribution of the surface layer. As follows from the graphs (a) and (b) in Fig. 13, it is the surface layer that contributes to the luminescence radiated well above the critical angle $\theta_c^{es}$ or, more precisely, above the threshold angle $\theta_{tr}$. Therefore, by defining the value of $\theta_{tr}$ and observing the angular distribution of the luminescence radiated into the (higher refractive index) substrate above $\theta_{tr}$, a detection technique which is the subject of the present invention can be established. Employing this technique, one can distinguish between the luminescence originating from the surface layer and the luminescence originating from the bulk covering the surface layer. The thickness of the surface layer has to be determined by the particular application exploiting this principle. Once it is known, the threshold angular position $\theta_{tr}$ can be calculated.

Using the technique of the present invention it is possible to distinguish between surface bound and bulk molecules which are luminescently labelled, which is of particular interest to biosensors. This has specific application in such biosensors in order to discriminate between surface-bound and bulk molecules which are luminescently labelled.

Using such an ability to distinguish the origin of the luminescence enables the present inventors to provide a method and technique which is adapted to enable detection of the luminescence originating from a region adjacent to the substrate interface and excited by a direct illumination. The thickness of the region of interest, which is in the order of the luminescence wavelength $\lambda$, can be tailored according to the needs of a particular application.

In the following analysis, the substrate is considered to be made of glass ($n_s$ = 1.515) and the environment containing the luminescent species is water ($n_w$ = 1.33). Similar conclusions can be, however, drawn for any other set of parameters, as will be apparent to those skilled in the art. As was detailed above the detection of the luminescence originating from a thin layer adjacent to the (glass) substrate, i.e., the surface layer, can be achieved by measuring a specific fraction of the luminescence, in particular that propagating in the (glass) substrate above the threshold angle $\theta_{tr}$. This conclusion is demonstrated in Fig. 13(b) where the difference between the angular profiles of the luminescence originating from the surface layer of thickness $t_s = \lambda$ (solid line) and the bulk layer of thickness $t_b = 3\lambda$ (dash-dotted line) are clearly visible. From the application point of view, it is important to know the relation between the thickness $t_s$ of the surface layer from which the luminescence originates and the threshold angle $\theta_{tr}$ above which the luminescence should be observed.

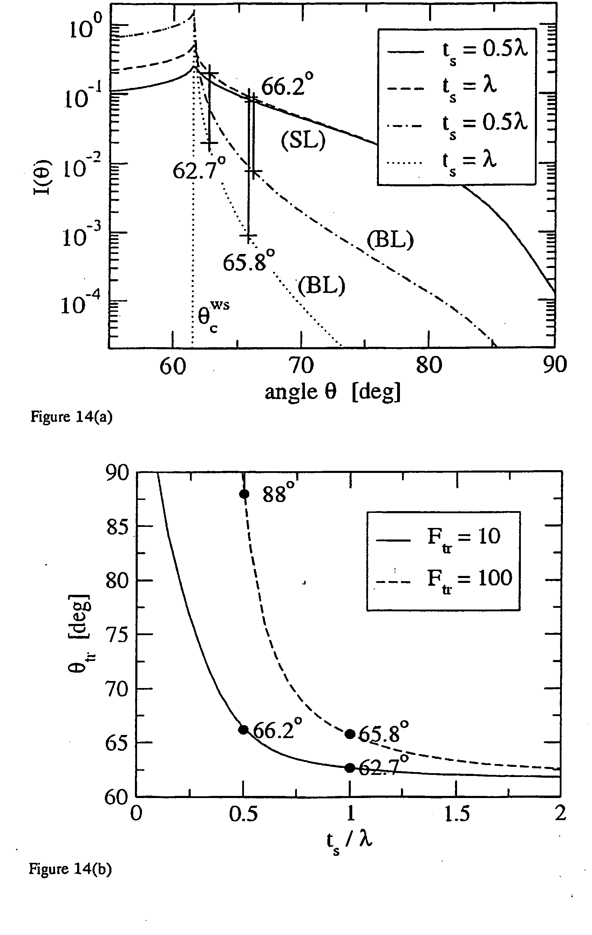

Figure 14(a) shows the angular distributions of the luminescence radiated from a 2-layer system depicted in Fig. 12. The distributions are plotted for two values of the surface layer thickness, namely $t_s$ = 0.5k and $t_s$ =$\lambda$, and for one value of the bulk layer thickness, namely $t_b = 3\lambda$. In order to see the relative relation between the various curves, the y-axis employs a logarithmic scale. From Fig. 14(a), it can be seen that there is a significant difference between the contributions to the luminescence originating from the surface and bulk layers, particularly above the critical angle $\theta_c^{ws}$ of the water/substrate (glass) interface. While the intensity corresponding to the bulk layer decreases abruptly for $\theta > \theta_c^{ws}$, this decrease is not so rapid for the intensity corresponding to the surface layer. Furthermore, the rate of this decrease varies with the thickness of the surface layer, which is the fundamental feature that can be exploited for determining the relation between $t_s$ and $\theta_{tr}$. The threshold angle $\theta_{tr}$ can be defined as the angle above which the ratio between the intensity of the luminescence originating from the surface layer ($I_s$) and that originating from the bulk layer ($I_b$) is greater than a specified threshold or performance factor $F_{tr}$: i.e.

$$I_s(\theta_{tr})/I_b(\theta_{tr}) > F_{tr}$$

Accordingly $\theta_{tr}$ can be defined as that angle which provides for the left-hand and right-hand sides of the above equation to be equal and the configurations developed in this application use the detection of light above this angle.

It will be appreciated that in most practical applications, the intensity of luminescence is always characterised by some non-zero background signal. These background signals may have contributions from electronic and other sources of noise, and represent a threshold value above which it is possible to detect a signal. Therefore, the value of $\theta_{tr}$ can be chosen in such a way that $I_b(\theta_{tr})$ within the above equation corresponds to this background level. Consequently, the definition above is simply a formal expression of the requirement that the signal-to-background ratio of the measurement of the luminescence originating from the surface layer be greater than some specified value $F_{tr}$. This also justifies the definition of $\theta_{tr}$ given by the equation. It can be seen from Fig. 14(a) that the intensity of the luminescence originating from the surface layer of thickness $t_s = \lambda$ is 10 times greater at $\theta_{tr} = 62.7°$ and 100 times greater at $\theta_{tr} = 65.8°$ than the intensity of the luminescence originating from the bulk layer. Therefore, by measuring the luminescence at angles $\theta \geq 62.7°$ and $\theta \geq 65.8°$, the certainty that only the luminescence originating from the surface layer of thickness $t_s =\lambda$ is measured is 10 and 100, respectively.

It will be appreciated that the choice of threshold angle is dependent on the configuration parameters but also on the confidence factor that is required by the user. This choice of threshold angle is provided by an analysis of the values of the threshold angles required to ensure that only the luminescence from within the surface layer is detected. As was detailed above this is provided by an examination of the values provided by the graphical output of Figure 14(b). The values provided by Figure 14(b) enable a calibration of the desired configuration. This calibration is provided based on an understanding of the parameters/factors contributing to the differentiation. This can be considered as a multi-step process:

1. The refractive indices of the materials involved in the particular application need to be known, namely $n_s$ of the substrate, $n_e$ of the environment and, optionally, $n_1$ of the thin surface layer, if different from $n_e$. These are specific to the configuration being used and can typically be found from the specification of the relevant materials and, in case of $n_e$, need to be either estimated or measured by other means.

2. The particular application will dictate the value of the thickness $t_1$ of the layer adjacent to the substrate/superstrate interface, i.e., the surface layer, which is of interest. This will also be defined by the application process and will be determined by the user.

3. The application will also dictate the so-called analytical wavelength $\lambda$ of luminescence, which is the wavelength where the luminescence intensity upon excitation by a certain light source is maximum.

4. Finally, the threshold factor $F_{tr}$ should be supplied by the user, which determines the level of confidence with which the luminescence originating from the surface layer and that originating from the surrounding bulk are to be distinguished.

5. From the values of $n_s$, $n_e$, $n_1$, $t_s$ and $\lambda$ supplied from steps 1-3, it is possible to defines a multilayer system such as that depicted in Figure 9 (the scenario where case $n_e$ is not equal to $n_1$) or in Figure 12 (the scenario where $n_e=n_1$). One also defines the value of the thickness $t_b$ of the bulk layer. This value is, in principle, an arbitrary value and greater than about $4\lambda$, but in practice it will be appreciated that the thickness of the bulk layer may be significantly greater than the thickness of the layer of interest.. This layer is utilised to determine an optimum threshold angle for the specific application and provides for an identification of the radiation originating from the bulk located above the surface layer.

6. In the following, it is assumed that $n_e$ and $n_1$ are not equal, but it will be apparent to the person skilled in the art that the same steps could be carried out if $n_e$ and $n_1$ are equal.

7. Utilising the techniques provided by the model described in the article (Polerecky et al, Applied Optics, 2000), or any equivalent model as will be appreciated by those skilled in the art, enables one to evaluate the angular distribution of luminescence intensity radiated from the molecules located in an arbitrary multilayer system.

8. Firstly, the situation in Fig. 9(a) is considered, i.e., the bulk layer is assumed to contain radiating molecules while the surface layer not. Using such a modeled set of parameters, the angular distribution of luminescence radiated from such a structure is calculated and a curve similar to the dash-dotted curve in Fig. 10(a) is obtained.

9. Secondly, the situation in Fig. 9(b) is considered, i.e., the surface layer is assumed to contain radiating molecules while the bulk layer not. Using the modeled parameters, the angular distribution of luminescence radiated from such a structure is calculated and a curve similar to the solid curve in Fig. 10(b) is obtained.

10. The curves corresponding to the calculations in points 9 and 8 are divided, i.e., the ratio between the intensities of the luminescence originating from the surface and bulk layers may then be calculated. Subsequently, the value of the angle at which this ratio is equal to the threshold factor $F_{tr}$ specified in point 4 is determined. This angle is equal to the threshold angle $\theta_{tr}$. This parameter can subsequently be used as the parameter defining the experimental conditions at which the method of this specification is used.

It will be appreciated that this initial definition of the optical properties of the system configuration enables one to perform a calculation of the angular distribution of the radiated luminescence. Once this distribution is calculated, the value of the thickness $t_1$ is chosen, according to the requirements of the application, the performance factor is chosen and then the threshold angle can immediately found.

As follows from the curves in Fig. 14(a) calculated for a different value of $t_s$, the value of the threshold angle $\theta_{tr}$ varies with the desired thickness of the surface layer. For example, $\theta_{tr} = 62.7°$ for $t_s = \lambda$ and $F_{tr} = 10$ but it increases to $\theta_{tr} = 66.2°$ for $t_s = 0.5\lambda$ and $F_{tr} = 10$. Therefore, from the practical application point of view, it is necessary to establish the relation between $t_s$ and the corresponding value of $\theta_{tr}$. Understandably, this relation is parameterised by the threshold factor $F_{tr}$. An example of $\theta_{tr}$ as a function of $t_s$ is shown in Fig. 14(b), where the solid and dashed lines correspond to $F_{tr} = 10$ and $F_{tr} = 100$, respectively. The graph implies that, for example, if an application requires that only the luminescence originating from a surface layer of thickness $t_s = 0.5\lambda$ and $t_s = \lambda$ be detected with a certainty characterised by $F_{tr} = 10$, the detector should measure only the luminescence radiated at angles greater than $\theta_{tr} = 66.2°$ and $\theta_{tr} = 62.7°$, respectively. These angles increase to approximately 88° and 65.8°, respectively, if a greater level of certainty, namely $F_{tr} = 100$, is required. The graph in Fig. 14(b) also shows that there are some limits with regard to the minimum thickness of the surface layer that can be resolved by this method. For example, the luminescence originating from a surface layer thinner than approximately $0.2\lambda$ cannot be detected with a certainty level of $F_{tr} \geq 10$, as indicated by the solid line which is not defined for $t_s < \approx 0.2\lambda$. This minimum thickness is increased to $\approx 0.5\lambda$ if the certainty level is increased to 100. This feature is related to the fact that the penetration depth of the evanescent field is greater than zero even for an incident angle approaching or equal to 90°. The graph also shows that if the surface region of thickness not exceeding $t_s = 2\lambda$ is of interest, it can be probed with a high certainty ($F_{tr} = 100$) by measuring the luminescence radiated above approximately 65°. This value is sufficiently small to be accessible by a simple experimental set-up, such as that detailed below.

It is important to emphasise that the excitation of luminescence was not mentioned in the above analysis at all. This is because the angular properties of the emitted luminescence, which are exploited in this technique, are independent of the way how the luminescent molecules are excited. Therefore, it is possible to use direct illumination for efficient excitation of the molecules while detecting the luminescence originating specifically from a close vicinity of the surface. This is what makes this technique very attractive.

It will be appreciated that application of the technique of the present invention enables one to extract information from areas where the luminescence of interest is that generated specifically by the molecules located in close vicinity to the surface. In particular, the present invention provides a method for the detection of such luminescence. In contrast to the conventional method that employs evanescent-wave excitation, this method enables one to use direct illumination to excite the luminescent molecules. The distinction between the luminescence radiated by molecules located in the bulk and near the surface is achieved by the measurement and appropriate treatment of the angular profile of luminescence intensity. In particular, by measuring the emitted luminescence above a certain threshold angle $\theta_{tr}$, only the luminescence originating from molecules located closer to the surface than some corresponding distance $t_s$ is detected. Taking into account that the excitation by direct illumination is much more efficient than that provided by the evanescent-wave excitation technique, for reasons including that more of the emitted light from the light source can be used for exciting the luminescence material, the method of the present application can be particularly attractive in immunosensing applications.

It will be appreciated that the above description identifies that, using the method of the present invention, it is possible to differentiate between the source of luminescence, i.e. that it is possible to discriminate between light originating directly or indirectly from a captured material or that originating from some spurious signal within a bulk layer, based on an angular orientation of the detector relative to the tagged material. The present invention however also provides for a modification of the substrate to which the tagged materials is optically coupled so as to enable the specific out-coupling of light radiated into the substrate at angles greater than the threshold angle to a suitably positioned detector.

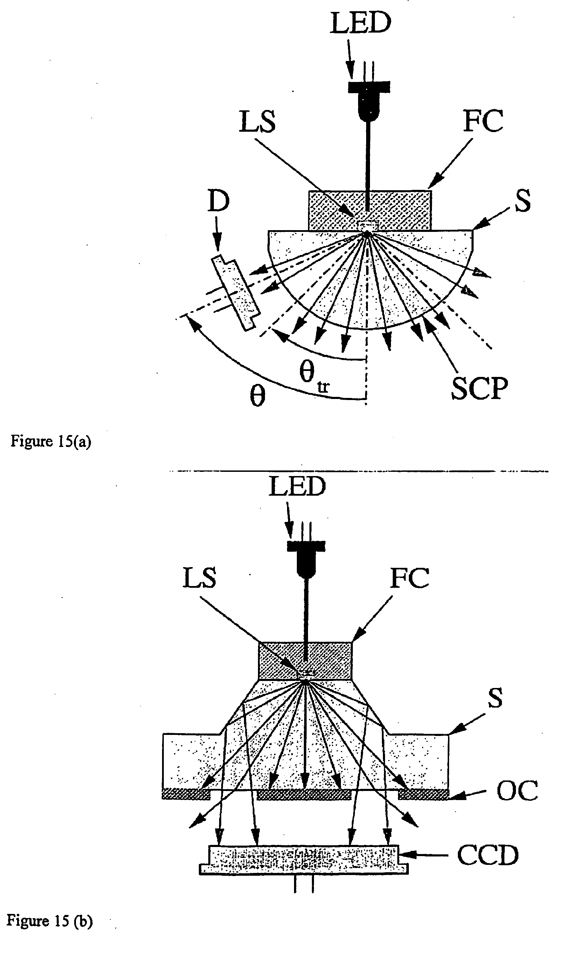

Figures 15a to 15d illustrate how a sensor configuration can be arranged so as to specifically outcouple the light of interest.

In the arrangement of Figure 15(a), the substrate (S), above which the luminescent solution can flow within a flow cell(FC), is attached to a semi-cylindrical prism (SCP) made from the same material as the substrate, for example glass. A luminescent sample (LS) is illuminated using a direct source of illumination such as a LED, and the resultant light propagating in the substrate above the threshold angle can be detected by a detector positioned at angles greater than the threshold angle. The detector is suitably a CCD camera, a linear detector array or some equivalent. Figures 15(b) to 15(d) show luminescent sensor configurations according to the present invention.

The embodiment of Figure 15 (b) employs a configuration utilising opaque coatings (OC), providing the substrate in the form of a frusto-conical configuration. The surface generated luminescence is transmitted to an area at the detector, which is spatially seperated from the area where the luminescence originating from the bulk layer is transmitted. This bulk layer contribution is occluded by providing opaque coatings which prevent the corresponding light from being detected or, again, by processing of the image obtained by the CCD chip, which acts as the detector.

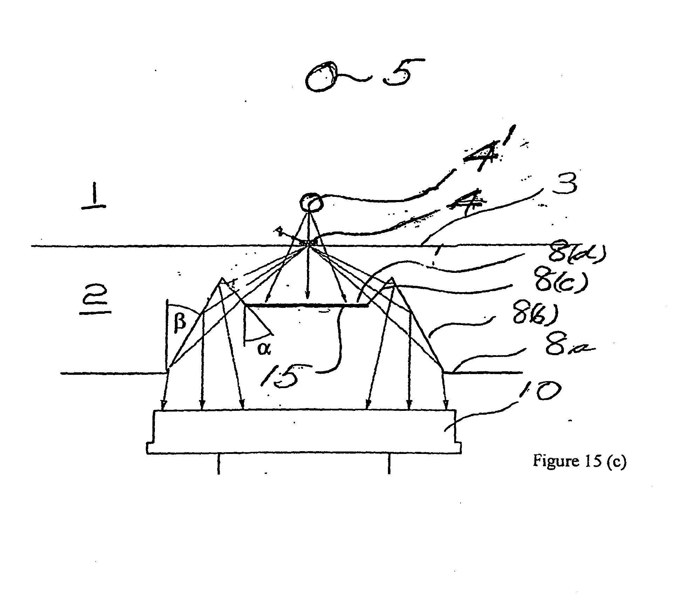

Figure 15(c) illustrates a construction of substrate, identified by the reference numeral 2, which is configured so as to provide such a selective out-coupling. In this embodiment, there is a lower configured surface 8, various parts of which are identified by the reference numerals 8(a), 8(b), 8(c) and 8(d). On the surface 8 (d), there is provided a light barrier 15 provided by an opaque surface formed on the lower surface 8(d) of the substrate 2. Thus, the luminescence generated in the superstrate 1 by the light source 5 exciting a luminescence source 4' in the superstrate 1 which is further away than some application specific distance $t_s$ (e.g. $t_s = 2\lambda$) (i.e. in the second layer) from the surface 3, will be absorbed by the opaque surface 8(d). However, the surface generated luminescence, that is to say, the luminescence generated by the light source 5 exciting the luminescent source 4 placed closer to the surfaces 8(c) and 8(b) and in the first layer, will be, by refraction and reflection, redirected to the detector 10. The light is first refracted through the surface 8(c) and then reflected downwards from the surface 8(b) towards the detector.

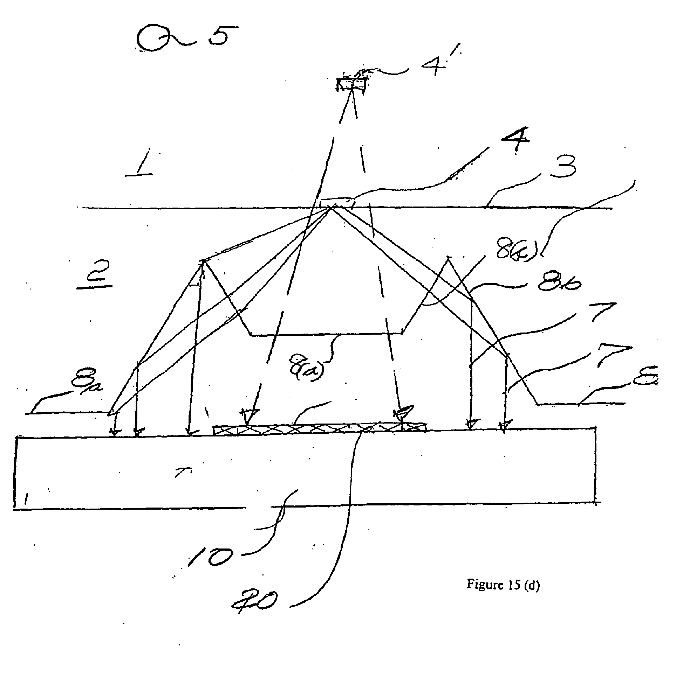

Referring now to Figure 15(d), there is illustrated an alternative construction of assembly, in which parts similar to those described with reference to Figure 15 are identified by the same reference numerals. In this embodiment, instead of placing any barrier on the substrate 2, the barrier is placed on the detector 10 and comprises a blocking plate 20 which, as can be seen, will block the luminescent light coming from the excitation of luminescent sources in the volume of the superstrate such as the luminescent source 4'.

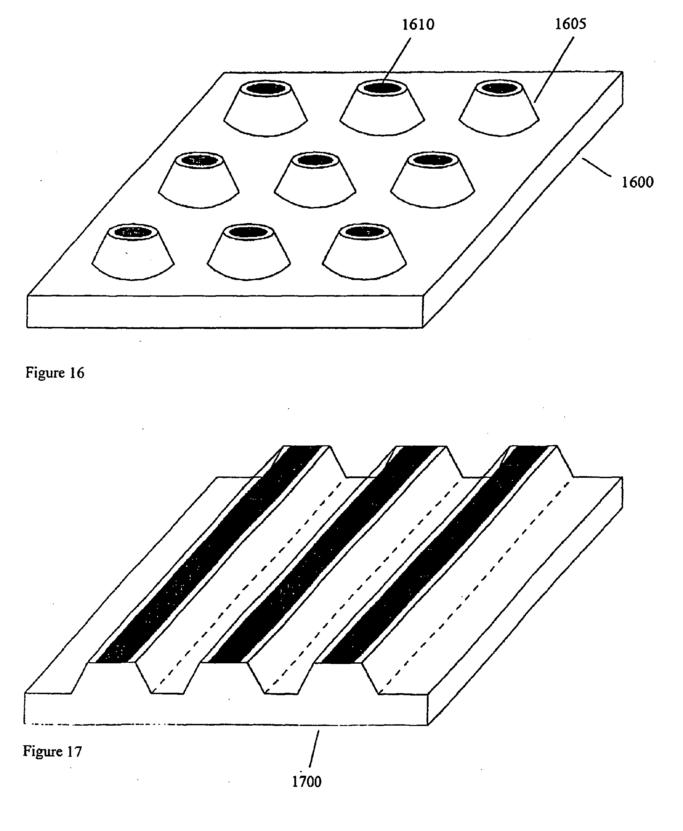

Figures 16 and 17 are schematic diagrams of sensor chips 1600, 1700 incorporating a plurality of individual sensor configurations. In Figure 16, a plurality of frusto-conical structures 1605, similar to that described in Figure 15b are deployed in an array. These frustrated cones each have a luminescent spot 1610 deposited on the upper surface thereof, and by suitably positioning a detector arrangement, the light emitting from each spot can be spatially distinguished. Figure 17 shows an alternative arrangement employing a groove or ridge like pattern.

Accordingly it will be appreciated that the present invention provides a technique for the collection of surface generated luminescence excited by direct illumination. The ability to discriminate by origin of the luminescence enables the use of such direct illumination, which is advantageous in that the sensitivity of sensor arrays utilising such techniques can be increased due to higher levels of illumination than hereintobefore possible.

In the specification the terms "comprise, comprises, comprised and comprising" or any variation thereof and the terms "include, includes, included and including" or any variation thereof are considered to be totally interchangeable and they should all be afforded the widest possible interpretation.

It will be further appreciated that the invention is not limited to the embodiments hereinbefore described but may be varied in both construction and detail. Furthermore, although certain embodiments may have been described with reference to specific integers or components it will be appreciated that individual components from different embodiments may be interchangeable depending on the desire of the user without departing from the scope of the present invention. It will ne further appreciated that although the present invention has been described with reference to specific substrate or medium ( fluid) types, that it is not intended to limit the present invention to these specific exemplary embodiments of the invention.

**Claims**

1. A luminescent sensor configuration for use in a medium

    (1) having a first refractive index and for discriminating between luminescent light emitted from a luminescent material provided in a first layer above a substrate (2) and that light emitted from a second layer above the substrate, the sensor configuration comprising:

        a) a source (5) of direct illumination,
        b) the substrate (2) having an upper (3) and lower (8) surface and being of a second refractive index,
        c) a material (4) capable of luminescence,
        d) a detector arrangement (10) provided below the lower surface of the substrate and adapted to detect light emitted through that lower surface,
        the luminescent sensor configuration being **characterised by**:
        e) a barrier (15, 20, OC) adapted to block light which has been transmitted into the substrate (2) at an angle below the critical angle from being detected by the detector arrangement (10),
        f) at least one optical redirection element at either an upper or lower surfaces of the substrate (2), the optical redirection element adapted to redirect light emitted by the luminescent material into the substrate at an angle greater than the critical angle out of the substrate and towards a detector

    and wherein, in use, the medium (1) and the substrate (2)
    (2) meet along the upper surface (3) of the substrate (2) which defines the boundary between the first and second refractive indices, the material (4) capable of luminescence is excited by the source of direct illumination (5), thereby luminescing and the detector arrangement (10) is adapted to discriminate between luminescent light emitted from a first layer within a predetermined distance of the upper surface (3) and light emitted from a second separate layer , the discrimination being effected by selective detection of light emitted from the luminescent material at angles greater than the critical angle of the medium/substrate interface.

2. The configuration as claimed in claim 1 wherein the predefined distance is less than about 4 $\lambda$, and preferably within the range of about 0.5 $\lambda$ to about 3 $\lambda$, wherein $\lambda$ is the wavelength of the luminescence light.

3. The configuration as claimed in claim 2 wherein the predefined distance is within the range of about 1 to about 2 $\lambda$.

4. The configuration as claimed in any preceding claim wherein the angle at which the luminescence is emitted into the substrate and subsequently selectively detected is greater than a threshold angle, the threshold angle being an angle which satisfies the equation:

$$I_s(\theta_{tr})/I_b(\theta_{tr}) = F_{tr,}$$

where $I_s(\theta_{tr})$ is the intensity of light emitted from the first layer at the threshold angle, $I_b(\theta_{tr})$ is the intensity of light emitted by the second layer at the threshold angle and $F_{tr}$ is a performance factor which is selected by the user.

5. The configuration as claimed in claim 4 wherein $I_b(\theta_{tr})$ corresponds to a background level within the configuration system such that the inequality reduces to providing a threshold angle which satisfies the inequality that the signal-to-background ratio of the measurement of the luminescence originating from the first layer is greater than some specified value $F_{tr}$.

6. The configuration as claimed in any preceding claim wherein the first and second layers have the same refractive index.

7. The configuration as claimed in any one of claims 1 to 5 wherein the first and second layers have a different refractive index.

8. The configuration as claimed in any preceding claim wherein the light emitted into the substrate is emitted from more than one source and the detector arrangement is adapted to spatially discriminate between the origins of the detected light.

9. The configuration as claimed in any preceding claim further comprising at least one portion of material adapted to capture a specific target species, the at least one portion of material being coupled to the substrate and adapted, in use, to capture any of a predefined target substance within the medium, the capture effecting the formation of a captured species, which either directly or indirectly is adapted to luminescence upon excitation, such luminescence being detectable by the detector.

10. The configuration as claimed in claim 9 comprising at least two distinct portions of material, each portion being coupled to the substrate and wherein the substrate is configured to redirect light emitted by each portion towards the detector such that the light received at the detector from a first portion is spatially independent from the light received at the detector from a second portion.

11. The configuration as claimed in any preceding claim wherein the light detected by the detector is not totally internally reflected within the substrate prior to detection.

12. The configuration as claimed in any preceding claim wherein the at least one optical redirection element is adapted to redirect the light using total internal reflection.

13. The sensor configuration as claimed in any preceding claim comprising a plurality of optical redirection elements (1605), each element comprises a frusto-conical structure raised above the upper surface of the substrate (1600), each frusto-conical structure having side walls and an upper surface, luminescent material (1610) being carried on the upper surface of the structure, and wherein light emitted by the material into the structure is internally reflected by the side walls of the structure and directed towards a detector positioned beneath the substrate.

14. The sensor configuration as claimed in any of claims 1 to 12 comprising a plurality of optical redirection elements, each element comprising a ridge raised above the upper surface of the substrate (1700) and extending along the upper surface of the substrate, the ridge having side walls and an upper surface, luminescent material being carried on the upper surface of the ridge, and wherein light emitted by the material into the ridge is internally reflected by the side walls of the ridge and directed towards a detector positioned beneath the substrate.

15. The sensor configuration as claimed in any of claims 1 to 11 wherein the at least one optical redirection element is adapted to redirect the light using refraction.

16. The sensor configuration as claimed in claim 15 wherein the at least one optical redirection element comprises a prism optically coupled to a lower surface of the substrate, the prism being adapted to receive light incident on the lower surface of the substrate and redirect that light sidewardly towards a detector.

17. The sensor configuration as claimed in claim 16 comprising a plurality of prisms each prism being associated with a unique spot on the upper surface of the substrate, such that light emitted by a spot is received within its associated prism and re-directed towards a detector.

18. The sensor configuration as claimed in claim 16 or claim 17 wherein the prism is optically coupled to the lower surface of the substrate and the prism has at least the same refractive index as the substrate to which it is optically coupled.

19. The sensor configuration as claimed in any of claims 1 to 11 wherein the at least one optical redirection element is adapted to redirect the light using diffraction.

20. The sensor configuration as claimed in claim 19 wherein the optical redirection element comprises a diffractive optical element provided at the lower surface of the substrate.

21. The sensor configuration as claimed in claim 1 wherein the lower surface of the substrate is structurally configured to both reflect and refract light radiated into the substrate, the reflection and refraction of the light effecting a redirection of light towards a detector, the light redirected being that light propagating into the substrate at an angle greater than the critical angle of the substrate/medium interface.

22. The sensor configuration as claimed in claim 21 wherein the structural configuration of the lower surface is such as to provide a first surface on which light emitted from the material and incident thereon is refracted out of the substrate and towards the second surface, which reflects the light which is incident thereon towards the detector.

23. The sensor configuration as claimed in claim 1 wherein the selective detection of light is effected by providing the substrate with non-parallel upper and lower surfaces, the angle of the upper and lower surfaces being such that the light emitted by the luminescence material is incident on the surfaces at angles greater than the critical angle of the substrate/medium interface, thereby effecting a propagation of light along an axis of the substrate towards a detector.

24. The sensor configuration as claimed in any preceding claim being further adapted to detect light radiated into the substrate by material in the first layer at angles which are not less than the critical angle of the material/substrate interface and greater than the critical angle of the medium/substrate interface.

25. The sensor configuration as claimed in any preceding claim wherein the detector is a CMOS, a CCD or a photodiode type detector.

26. A sensor configuration as claimed in any preceding claim wherein the material capable of luminescence is sensitive to an analyte with which the sensor is intended to be used, such that the presenca of an analyte in the medium with which the sensor is used, and the subsequent illumination or the configuration, effects a luminescence of the material, said luminescence being detectable at the detector.

27. A sensor configuration as claimed in any preceding claim wherein the sensor is provided initially with a bio-recognition element, the bio-recognition element being sensitive to and adapted to couple with any predefined target biological sample in the medium with which the sensor is used, and once coupled a further coupling of the coupled biological sample/bio-recognition element with a luminescent tag effects the formation of the luminescent material.

28. A sensor configuration as claimed in any preceding claim wherein the barrier (15, OC) is provided in or on the substrate (2).

29. A sensor configuration as claimed in any of claims 1 to 27 wherein the barrier (20) is provided on the detector (10).

30. A method of discriminating between luminescent light emitted from a luminescent material provided in a first layer above a substrate and that light emitted from a second layer above the substrate, the method comprising the steps of:

   a) illuminating the first and second layers with a source of direct illumination,
   b) providing a detector arrangement below the substrate,
   the method **characterised by**:
   c) arranging the detector and/or substrate so as to selectively discriminate between the sources of light detected, the discrimination being effected based on the angles at which the light propagates into the substrate, such that light emitted from the first layer only is detected at the detector,
   d) optically redirecting, at either an upper or lower surfaces of the substrate, light emitted by the luminescent material into the substrate at an angle greater than the critical angle out of the substrate and towards a detector, and
   e) blocking light which has been transmitted into the substrate at an angle below the critical angle from being detected by the detector arrangement.

31. The method as claimed in claim 30 wherein the angle is greater than the critical angle of an interface between the second layer and the substrate.

32. The method as claimed in any one of claims 30 or 31 wherein the angle is greater than a threshold angle, the threshold angle being that angle which satisfies the equation:

$$I_s(\theta_{tr}) / I_b(\theta_{tr}) = F_{tr}.$$

where $I_s(\theta_{tr})$ is the intensity of light emitted from the first layer at the threshold angle, $I_b(\theta_{tr})$ is the intensity of light emitted by the second layer at the threshold angle and $F_{tr}$ is a performance factor which is selected by the user.

33. The configuration as claimed in claim 32 wherein $I_b(\theta_{tr})$ corresponds to a background level within the configuration system such that the inequality reduces to providing a threshold angle which satisfies the inequality that the signal-to-background ratio of the measurement of the luminescence originating from the first layer is greater than some specified value $F_{tr}$.

**Patentansprüche**

1. Eine Lumineszenz-Sensorkonfiguration zur Verwendung in einem Medium

   (1) mit einem ersten Brechungsindex und zum Unterscheiden zwischen Lumineszenzlicht, das von einem lumineszierenden Material emittiert wird, das in einer ersten Schicht oberhalb eines Substrats (2) vorgesehen ist, und Licht, das von einer zweiten Schicht oberhalb des Substrats emittiert wird, wobei die Sensorkonfiguration Folgendes aufweist:

   a) eine Quelle (5) direkter Illumination,
   b) das Substrat (2) mit einer oberen (3) und einer unteren (8) Seite, wobei das Substrat einen zweiten Brechungsindex aufweist,

c) ein Material (4), das in der Lage ist zu leuchten bzw. zu lumineszieren,

d) eine Detektoranordnung (10), die unterhalb der Unterseite des Substrats vorgesehen ist, und die in der Lage ist Licht zu detektieren, das durch die Unterseite emittiert wird,

wobei die Lumineszenz-Sensorkonfiguration **gekennzeichnet ist durch**:

e) eine Barriere (15, 20, OC), die in der Lage ist Licht gegenüber einer Detektierung **durch** die Detektoranordnung (10) zu blokkieren, das in das Substrat (2) unter einem Winkel unterhalb des kritischen Winkels in das Substrat (2) gesendet wurde,

f) wenigstens ein optisches Umlenkelement an entweder einer Ober-oder Unterseite des Substrats (2), wobei das optische Umlenkelement in der Lage ist, Licht das **durch** das lumineszierende Material in das Substrat mit einem Winkel größer als dem kritischen Winkel aus dem Substrat heraus umzulenken, und zwar zu einem Detektor, und wobei sich bei der Verwendung das Medium (1) und das Substrat (2) entlang der Oberseite (3) des Substrats (2) treffen, was die Grenzfläche zwischen den ersten und zweiten Brechungsindices bildet, wobei das Material (4), das in der Lage ist zu leuchten, **durch** die Quelle direkter Illumination (5) erregt wird, um **dadurch** zu leuchten und wobei die Detektoranordnung (10) in der Lage ist, zwischen Lumineszenzlicht, das von einer ersten Schicht innerhalb eines vorbestimmten Abstands von der Oberseite (3) und Licht, das von einer zweiten separaten Schicht emittiert wurde zu unterscheiden, wobei die Unterscheidung bewirkt wird **durch** selektive Detektierung von Licht, das von dem lumineszierenden Material mit Winkeln emittiert wird, die größer sind als der kritische Winkel der Medium/Substrat-Zwischenfläche.

2. Konfiguration nach Anspruch 1, wobei der vorbestimmte Abstand kleiner ist als ungefähr 4 λ und vorzugsweise innerhalb des Bereichs von ungefähr 0,5 λ bis ungefähr 3 λ liegt, wobei λ die Wellenlänge des Lumineszenzlichtes ist.

3. Konfiguration nach Anspruch 2, wobei der vorbestimmte Abstand innerhalb des Bereichs von ungefähr 1 bis ungefähr 2 λ liegt.

4. Konfiguration nach einem der vorhergehenden Ansprüche, wobei der Winkel mit dem das Lumineszenzlicht in das Substrat emittiert und nachfolgend selektiv detektiert wird, größer ist als ein Schwellenwertwinkel, wobei der Schwellenwertwinkel ein Winkel ist, der die folgende Gleichung erfüllt:

$$I_s(\theta_{tr}) \, / \, I_b \, (\theta_{tr}) = F_{tr},$$

wobei $I_s(\theta_{tr})$ die Intensität des von der ersten Schicht mit dem Schwellenwertwinkel emittierten Lichtes ist, $I_b \, (\theta_{tr})$ die Intensität des durch die zweite Schicht mit dem Schwellenwertwinkel emittierten Lichtes ist, und $F_{tr}$ ein Leistungsfaktor ist, der durch den Nutzer ausgewählt ist.

5. Konfiguration nach Anspruch 4, wobei $I_b \, (\theta_{tr})$ einem Hintergrundniveau innerhalb des Konfigurationssystems ist, so dass die Ungleichung sich darauf reduziert einen Schwellenwertwinkel vorzusehen, der die Ungleichung erfüllt, so dass das Signal-zu-Hintergrund-Verhältnis der Messung der Lumineszenz, die von der ersten Schicht stammt, größer ist als ein vorgegebener Wert $F_{tr}$.

6. Konfiguration nach einem der vorhergehenden Ansprüche, wobei die ersten und zweiten Schichten denselben Brechungsindex besitzen.

7. Konfiguration nach einem der Ansprüche 1 bis 5, wobei die ersten und zweiten Schichten einen unterschiedlichen Brechungsindex besitzen.

8. Konfiguration nach einem der vorhergehenden Ansprüche, wobei das Licht das in das Substrat emittiert wird, von mehr als einer Quelle emittiert wird, und die Detektoranordnung in der Lage ist, räumlich zwischen den Ursprüngen des detektierten Lichtes zu unterscheiden.

9. Konfiguration nach einem der vorhergehenden Ansprüche, die ferner wenigstens einen Materialteil aufweist, der in der Lage ist ein spezifisches Target-Spezies einzufangen, wobei der wenigstens eine Materialteil mit dem Substrat gekoppelt und in der Lage ist, während der Verwendung irgendeine vordefinierte Target-Substanz innerhalb des Mediums einzufangen, wobei das Einfangen die Formation einer eingefangenen Spezies bewirkt, die entweder direkt oder indirekt in der Lage ist, bei Erregung zu lumineszieren, wobei eine solche Lumineszenz durch den Detektor detektierbar ist.

10. Konfiguration nach Anspruch 9, die wenigstens zwei distinkte Materialteile aufweist, wobei jeder Teil mit dem Substrat gekoppelt ist, und wobei das Substrat konfiguriert ist Licht umzulenken, das durch jeden der Teile emittiert wird, und zwar zu dem Detektor, so dass das Licht das an dem Detektor von einem ersten Teil empfangen wird, räumlich unabhängig ist von dem Licht, das an dem Detektor von einem zweiten Teil empfangen wird.

11. Konfiguration nach einem der vorhergehenden Ansprüche, wobei das durch den Detektor detektierte Licht vor seiner Detektierung nicht vollständig intern innerhalb des Substrats reflektiert wird.

12. Konfiguration nach einem der vorhergehenden Ansprüche, wobei das wenigstens eine optische Umlenkelement in der Lage ist, das Licht unter Verwendung vollständiger interner Reflektion umzulenken.

13. Sensorkonfiguration nach einem der vorhergehenden Ansprüche, die Folgendes aufweist: eine Vielzahl von optischen Umlenkelementen (1605), wobei jedes Element eine kegelstumpfförmige Struktur aufweist, die über die Oberseite des Substrats (1600) angehoben ist, wobei jede kegelstumpfförmige Struktur Seitenwände und eine Oberseite besitzt, lumineszierendes Material (1610) das auf der Oberseite der Struktur getragen wird, und wobei Licht, das durch das Material in die Struktur emittiert wird, intern durch die Seitenwände der Struktur reflektiert wird, und zu einem Detektor gerichtet wird, der unterhalb des Substrats positioniert ist.

14. Sensorkonfiguration nach einem der Ansprüche 1 bis 12, die Folgendes aufweist: eine Vielzahl von optischen Umlenkelementen, wobei jedes Element eine Rippe bzw. Kante aufweist, die sich über die Oberseite des Substrats (1700) erhebt, und sich entlang der Oberseite des Substrats erstreckt, wobei die Rippe Seitenwände und eine Oberseite besitzt, lumineszierendes Material, das auf der Oberseite der Rippe getragen wird, und wobei Licht, das durch das Material in die Rippe emittiert wird, intern durch die Seitenwände der Rippe reflektiert wird, und zu einem Detektor gerichtet wird, der unterhalb des Substrats positioniert ist.

15. Sensorkonfiguration nach einem der Ansprüche 1 bis 11, wobei das wenigstens eine optische Umlenkelement in der Lage ist, das Licht unter Verwendung von Brechung umzulenken.

16. Sensorkonfiguration nach Anspruch 15, wobei das wenigstens eine optische Umlenkelement ein Prisma aufweist, das optisch mit einer Unterseite des Substrats gekoppelt ist, wobei das Prisma in der Lage ist Licht zu empfangen, das auf die Unterseite des Substrats einfällt um dieses Licht seitlich zu einem Detektor umzulenken.

17. Sensorkonfiguration nach Anspruch 16, die eine Vielzahl von Prismen aufweist, wobei jedes Prisma mit einem einzigartigen Punkt auf der Oberseite des Substrats assoziiert ist, so dass Licht das durch einen Punkt emittiert wird, innerhalb des assoziierten Prismas empfangen und zu einem Detektor umgelenkt wird.

18. Sensorkonfiguration nach Anspruch 16 oder 17, wobei das Prisma optisch mit der Unterseite des Substrats gekoppelt ist und das Prisma wenigstens denselben Brechungsindex besitzt wie das Substrat mit dem es optisch gekoppelt ist.

19. Sensorkonfiguration nach einem der Ansprüche 1 bis 11, wobei das wenigstens eine optische Umlenkelement in der Lage ist das Licht unter Verwendung von Beugung umzulenken.

20. Sensorkonfiguration nach Anspruch 19, wobei das optische Umlenkelement ein beugendes optisches Element aufweist, das an der Unterseite des Substrats vorgesehen ist.

21. Sensorkonfiguration nach Anspruch 1, wobei die Unterseite des Substrats eine solche strukturelle Konfiguration aufweist, dass in das Substrat eingestrahltes Licht sowohl reflektiert als auch gebrochen wird, wobei die Reflektion und Brechung des Lichtes eine Umlenkung des Lichtes zu einem Detektor bewirkt, wobei das umgelenkte Licht das Licht ist, das in das Substrat mit einem Winkel der größer ist als der kritische Winkel der Substrat/Medium-Zwischenfläche fortpflanzt.

22. Sensorkonfiguration nach Anspruch 21, wobei die strukturelle Konfiguration an der Unterseite derart ausgebildet ist, dass sie eine erste Oberfläche vorsieht, an der von dem Material emittiertes und darauf einfallendes Licht durch Brechung aus dem Substrat heraus und zu der zweiten Oberfläche geleitet wird, welche das darauf einfallende Licht zu dem Detektor reflektiert.

23. Sensorkonfiguration nach Anspruch 1, wobei die selektive Detektierung von Licht bewirkt wird durch Versehen des Substrats mit nicht-parallelen Ober- und Unterseiten, wobei der Winkel zwischen den Ober- und Unterseiten derart gewählt ist, dass durch das lumineszierende Material emittiertes Licht auf den Oberflächen mit Winkeln auftritt, die größer als der kritische Winkel der Substrat/Medium-Zwischenfläche ist, um **dadurch** eine Fortpflanzung von Licht entlang einer Achse des Substrats zu einem Detektor zu bewirken.

24. Sensorkonfiguration nach einem der vorhergehenden Ansprüche, die ferner in der Lage ist Licht zu detektieren, das in das Substrat eingestrahlt wird durch Material in der ersten Schicht und zwar mit Winkeln die nicht kleiner sind als der kritische Winkel der Material/Substrat-Zwischenfläche und größer als der kritische Winkel der Medium/Substrat-Zwischenfläche.

**25.** Sensorkonfiguration nach einem der vorhergehenden Ansprüche, wobei der Detektor ein CMOS oder CCD oder ein Detektor des Fotodioden-Typs ist.

**26.** Sensorkonfiguration nach einem der vorhergehenden Ansprüche, wobei das Material, das in der Lage ist zu lumineszieren, gegenüber einem Analyt empfindlich ist, mit dem der Sensor verwendet werden soll, so dass die Gegenwart eines Analyts in dem Medium mit dem der Sensor verwendet wird, und die nachfolgende Beleuchtung der Konfiguration eine Lumineszenz des Materials bewirkt, wobei die Lumineszenz an dem Detektor detektierbar ist.

**27.** Sensorkonfiguration nach einem der vorhergehenden Ansprüche, wobei der Sensor anfänglich mit einem Bio-Erkennungselement versehen ist, wobei das Bio-Erkennungselement empfindlich ist für irgendeine vorbestimmte biologische Targetprobe in dem Medium mit dem der Sensor verwendet wird, und in der Lage ist mit diesem zu koppeln, und wobei nach einer Kopplung eine weitere Kopplung der Kopplung aus biologischer Probe/Bio-Erkennungselement mit einem Lumineszenz-Tag die Bildung des lumineszierenden Materials bewirkt.

**28.** Sensorkonfiguration nach einem der vorhergehenden Ansprüche, wobei die Barriere (15, OC) in oder an dem Substrat (2) vorgesehen ist.

**29.** Sensorkonfiguration nach einem der Ansprüche 1 bis 27, wobei die Barriere (20) an dem Detektor (10) vorgesehen ist.

**30.** Verfahren zum Unterscheiden zwischen Lumineszenzlicht, das von einem lumineszierenden Material emittiert wird, das in einer ersten Schicht oberhalb eines Substrats vorgesehen ist, und von Licht das von einer zweiten Schicht oberhalb des Substrats emittiert wird, wobei das Verfahren die folgenden Schritte aufweist:

a) Beleuchten der ersten und zweiten Schichten mit einer Quelle direkter Illumination,
b) Vorsehen einer Detektoranordnung unterhalb des Substrats, wobei das Verfahren **gekennzeichnet ist durch** die folgenden Schritte:
c) Anordnen des Detektors und/oder Substrats zum selektiven Unterscheiden zwischen den Quellen von detektiertem Licht, wobei die Unterscheidung bewirkt wird basierend auf den Winkeln, mit denen sich das Licht in das Substrat fortpflanzt, so dass nur Licht das von der ersten Schicht emittiert wird, an dem Detektor detektiert wird,
d) optisches Umlenken an entweder einer Ober- oder Unterseite des Substrats von Licht, das **durch** das lumineszierende Material in das Substrat mit einem Winkel größer als dem kritischen Winkel emittiert wurde, und zwar aus dem Substrat heraus und zu einem Detektor, und
e) Blockieren von Licht, das in das Substrat mit einem Winkel unterhalb des kritischen Winkels gesendet wurde, gegenüber einer Detektierung **durch** die Detektoranordnung.

**31.** Verfahren nach Anspruch 30, wobei der Winkel größer ist als der kritische Winkel einer Zwischenfläche zwischen der zweiten Schicht und dem Substrat.

**32.** Verfahren nach einem der Ansprüche 30 oder 31, wobei der Winkel größer ist als ein Schwellenwertwinkel, wobei der Schwellenwertwinkel der Winkel ist, der die folgende Gleichung erfüllt:

$$I_s(\theta_{tr}) \, / \, I_b \, (\theta_{tr}) = F_{tr},$$

wobei $I_s(\theta_{tr})$ die Intensität des von der ersten Schicht mit dem Schwellenwertwinkel emittierten Lichtes ist, $I_b(\theta_{tr})$ die Intensität des durch die zweite Schicht mit dem Schwellenwertwinkel emittierten Lichtes ist, und $F_{tr}$ ein Leistungsfaktor ist, der durch den Nutzer ausgewählt ist.

**33.** Konfiguration nach Anspruch 32, wobei $I_b(\theta_{tr})$ einem Hintergrundlevel innerhalb des Konfigurationssystems entspricht, so dass die Ungleichung sich darauf reduziert einen Schwellenwertwinkel vorzusehen, der die Ungleichung erfüllt, so dass das Signal-zu-Hintergrund-Verhältnis der Messung der Lumineszenz die von der ersten Schicht stammt, größer ist als ein spezifizierter Wert $F_{tr}$.

**Revendications**

**1.** Agencement de capteur reposant sur la luminescence pour utilisation dans un milieu (1) ayant un premier indice de réfraction et pour faire une discrimination entre de la lumière émise par luminescence par un matériau luminescent prévu dans une première couche au-dessus d'un substrat (2) et cette lumière émise à partir d'une deuxième couche au-dessus de la surface, l'agencement de capteur comprenant :

a) une source (5) d'éclairage direct,
b) le substrat (2) comprenant une surface supérieure (3) et une surface inférieure (8) et ayant un deuxième indice de réfraction,
c) un matériau (4) capable de luminescence,

d) un agencement de détecteur (10) prévu sous la surface inférieure du substrat et apte à détecter de la lumière émise à travers cette surface inférieure,

l'agencement de capteur reposant sur la luminescence étant **caractérisé par** :

e) une barrière (15, 20, OC) apte à bloquer de la lumière qui a été transmise dans le substrat (2) avec un angle inférieur à l'angle critique et l'empêcher d'être détectée par l'agencement de détecteur (10),

f) au moins un élément de redirection optique au niveau d'une surface supérieure ou d'une surface inférieure du substrat (2), l'élément de redirection optique étant apte à rediriger de la lumière émise par le matériau luminescent dans le substrat avec un angle supérieur à l'angle critique hors du substrat en direction d'un détecteur

et dans lequel, en utilisation, le milieu (1) et le substrat (2) se rejoignent le long de la surface supérieure (3) du substrat (2) qui définit la frontière entre les premier et deuxième indices de réfraction, le matériau (4) capable de luminescence est excité par la source d'éclairage direct (5), devenant ainsi luminescent, et l'agencement de détecteur (10) est apte à faire une discrimination entre de la lumière de luminescence émise à partir d'une première couche se trouvant dans une limite de distance prédéterminée par rapport à la surface supérieure (3) et de la lumière émise à partir d'une deuxième couche séparée, la discrimination étant effectuée par une détection sélective de lumière émise par le matériau luminescent à des angles supérieurs à l'angle critique de l'interface milieu/substrat.

2. Agencement selon la revendication 1, dans lequel la distance prédéfinie est inférieure à environ 4 $\lambda$, et de préférence dans la plage comprise entre 0,5 $\lambda$ et environ 3 $\lambda$, $\lambda$ étant la longueur d'onde de la lumière de luminescence.

3. Agencement selon la revendication 2, dans lequel la distance prédéfinie est dans la plage comprise entre environ 1 et environ 2 $\lambda$.

4. Agencement selon l'une quelconque des revendications précédentes, dans lequel l'angle avec lequel la luminescence est émise dans le substrat et ensuite détectée sélectivement est supérieur à un angle de seuil, l'angle de seuil étant un angle qui satisfait l'équation :

$$I_S(\theta_{tr})/I_b(\theta_{tr}) = F_{tr},$$

où $I_s(\theta_{tr})$ est l'intensité de la lumière émise à partir de la première couche à l'angle de seuil, $I_b(\theta_{tr})$ est l'intensité de la lumière émise à partir de la deuxième couche à l'angle de seuil et $F_{tr}$ est un facteur de performance qui est choisi par l'utilisateur.

5. Agencement selon la revendication 4, dans lequel $I_b(\theta_{tr})$ correspond à un niveau de fond dans le système d'agencement de sorte que l'inégalité se réduit à prévoir un angle de seuil qui satisfait l'inégalité consistant en ce que le rapport signal-sur-fond de la mesure de la luminescence provenant de la première couche est supérieur à une certaine valeur spécifiée de $F_{tr}$.

6. Agencement selon l'une quelconque des revendications précédentes, dans lequel les première et deuxième couches ont le même indice de réfraction.

7. Agencement selon l'une quelconque des revendications 1 à 5, dans lequel les première et deuxième couches ont des indices de réfraction différents.

8. Agencement selon l'une quelconque des revendications précédentes, dans lequel la lumière émise dans le substrat est émise par plusieurs sources et l'agencement de détecteur est apte à discriminer spatialement les origines de la lumière détectée.

9. Agencement selon l'une quelconque des revendications précédentes, comprenant en outre au moins une partie du matériau apte à capturer une espèce cible spécifique, ladite au moins une partie du matériau étant couplée au substrat et apte, en utilisation, à capturer toute substance cible prédéfinie dans le milieu, la capture réalisant la formation d'une espèce capturée, qui directement ou indirectement est apte à être luminescente lors d'une excitation, cette luminescence étant détectable par le détecteur.

10. Agencement selon la revendication 9, comprenant au moins deux parties distinctes de matériau, chaque partie étant couplée au substrat et dans lequel le substrat est agencé pour rediriger de la lumière émise par chaque partie vers le détecteur de sorte que la lumière reçue au niveau du détecteur à partir d'une première partie est spatialement indépendante de la lumière reçue au niveau du détecteur à partir d'une deuxième partie.

11. Agencement selon l'une quelconque des revendications précédentes, dans lequel la lumière détectée par le détecteur n'est pas entièrement réfléchie de façon interne dans le substrat avant la détection.

12. Agencement selon l'une quelconque des revendications précédentes, dans lequel ledit au moins un élément de redirection optique est apte à rediriger la lumière en utilisant une réflexion interne totale.

13. Agencement de capteur selon l'une quelconque des revendications précédentes, comprenant une pluralité d'éléments de redirection optique (1605), chaque élément comprenant une structure tronconique élevée au-dessus de la surface supérieure du substrat (1600), chaque structure tronconique comportant des parois latérales et une surface supérieure, un matériau luminescent (1610) étant porté par la surface supérieure de la structure, et dans lequel de la lumière émise par le matériau dans la structure est réfléchie de façon interne par les parois latérales de la structure et dirigée vers un détecteur positionné en dessous du substrat.

14. Agencement de capteur selon l'une quelconque des revendications 1 à 12, comprenant une pluralité d'éléments de redirection optique, chaque élément comprenant une arrête élevée au-dessus de la surface supérieure du substrat (1700) et s'étendant le long de la surface supérieure du substrat, l'arrête comportant des parois latérales et une surface supérieure, un matériau luminescent étant porté par la surface supérieure de l'arrête, et dans lequel de la lumière émise par le matériau dans l'arrête est réfléchie de façon interne par les parois latérales de l'arrête et dirigée vers un détecteur positionné en dessous du substrat.

15. Agencement de capteur selon l'une quelconque des revendications 1 à 11, dans lequel ledit au moins un élément de redirection optique est apte à rediriger la lumière en utilisant une réfraction.

16. Agencement de capteur selon la revendication 15, dans lequel ledit au moins un élément de redirection optique comprend un prisme couplé optiquement à une surface inférieure du substrat, le prisme étant apte à recevoir de la lumière incidente sur la surface inférieure du substrat et à rediriger cette lumière latéralement vers un détecteur.

17. Agencement de capteur selon la revendication 16, comprenant une pluralité de prismes, chaque prisme étant associé à une zone ponctuelle unique de la surface supérieure du substrat, de sorte que la lumière émise par une zone ponctuelle est reçue dans son prisme associé et est redirigée vers un détecteur.

18. Agencement de capteur selon la revendication 16 ou 17, dans lequel le prisme est couplé optiquement à la surface inférieure du substrat et le prisme a au moins le même indice de réfraction que le substrat auquel il est couplé optiquement.

19. Agencement de capteur selon l'une quelconque des revendications 1 à 11, dans lequel ledit au moins un élément de redirection optique est apte à rediriger la lumière en utilisant une diffraction.

20. Agencement optique selon la revendication 19, dans lequel l'élément de redirection optique comprend un élément optique diffractant prévu au niveau de la surface inférieure du substrat.

21. Agencement de capteur selon la revendication 1, dans lequel la surface inférieure du substrat est structurellement agencée pour refléter et réfracter à la fois de la lumière rayonnée dans le substrat, la réflexion et la réfraction de la lumière réalisant une redirection de lumière vers un détecteur, la lumière redirigée étant cette lumière se propageant dans le substrat avec un angle supérieur à l'angle critique de l'interface substrat/milieu.

22. Agencement de capteur selon la revendication 21, dans lequel l'agencement structurel de la surface inférieure est tel qu'il fournit une première surface sur laquelle de la lumière émise par le matériau et incidente sur celle-ci est réfractée hors du substrat et en direction de la deuxième surface, qui réfléchit la lumière qui est incidente sur celle-ci vers le détecteur.

23. Agencement de capteur selon la revendication 1, dans lequel la détection sélective de lumière est réalisée en prévoyant le substrat avec des surfaces supérieure et inférieure non parallèles, l'angle entre les surfaces supérieure et inférieure étant tel que la lumière émise par le matériau de luminescence est incidente sur les surfaces avec des angles supérieurs à l'angle critique de l'interface substrat/milieu, réalisant ainsi une propagation de lumière suivant un axe du substrat vers un détecteur.

24. Agencement de capteur selon l'une quelconque des revendications précédentes, étant en outre apte à détecter de la lumière rayonnée dans le substrat par un matériau situé dans la première couche avec des angles qui ne sont pas inférieurs à l'angle critique de l'interface matériau/substrat et supérieurs à l'angle critique de l'interface milieu/substrat.

25. Agencement de capteur selon l'une quelconque des revendications précédentes, dans lequel le détecteur est un détecteur de type CMOS, CCD ou à photodiode.

26. Agencement de capteur selon l'une quelconque des revendications précédentes, dans lequel le matériau capable de luminescence est sensible à un analyte avec lequel le capteur est destiné à être utilisé, de sorte que la présence d'un analyte dans le milieu avec lequel le support est utilisé, et l'éclairage ultérieur de l'agencement, entraînent une luminescence du matériau, la luminescence étant détectable au niveau du détecteur.

27. Agencement de capteur selon l'une quelconque des revendications précédentes, dans lequel le capteur est muni initialement d'un élément de bio-reconnaissance, l'élément de bio-reconnaissance étant sensible et apte à un couplage avec tout échantillon biologique cible prédéfini dans le milieu avec lequel le capteur est utilisé, et après le couplage, un autre couplage de l'échantillon biologique/élément de bio-reconnaissance couplés avec une étiquette luminescente entraîne la formation du matériau luminescent.

28. Agencement de capteur selon l'une quelconque des revendications précédentes, dans lequel la barrière (15, OC) est prévue dans ou sur le substrat (2).

29. Agencement de capteur selon l'une quelconque des revendications 1 à 27, dans lequel la barrière (20) est prévue sur le détecteur (10).

30. Procédé de discrimination entre de la lumière luminescente émise à partir d'un matériau luminescent prévu dans une première couche au-dessus d'un substrat et cette lumière émise à partir d'une deuxième couche au-dessus du substrat, le procédé comprenant les étapes suivantes :

    a) éclairer les première et deuxième couches avec une source d'éclairage direct,
    b) prévoir un agencement de détecteur en dessous du substrat,
    le procédé étant **caractérisé par** :
    c) agencer le détecteur et/ou le substrat de façon à discriminer sélectivement les sources de lumière détectées, la discrimination étant effectuée en fonction des angles avec lesquels la lumière se propage dans le substrat, de sorte que cette lumière émise par la première couche seulement est détectée au niveau du détecteur,
    d) rediriger optiquement, au niveau d'une surface supérieure ou d'une surface inférieure du substrat, de la lumière émise par le matériau luminescent dans le substrat avec un angle supérieur à l'angle critique hors du substrat et en direction d'un détecteur, et
    e) bloquer de la lumière qui a été transmise dans le substrat avec un angle inférieur à l'angle critique pour l'empêcher d'être détectée par l'agencement de détecteur.

31. Procédé selon la revendication 30, dans lequel l'angle est supérieur à l'angle critique d'une interface entre la deuxième couche et le substrat.

32. Procédé selon l'une quelconque des revendications 30 ou 31, dans lequel l'angle est supérieur à l'angle de seuil, l'angle de seuil étant l'angle qui satisfait l'équation :

$$I_S(\theta_{tr})/I_b(\theta_{tr}) = F_{tr},$$

où $I_S(\theta_{tr})$ est l'intensité de la lumière émise à partir de la première couche à l'angle de seuil, $I_b(\theta_{tr})$ est l'intensité de la lumière émise à partir de la deuxième couche à l'angle de seuil et $F_{tr}$ est un facteur de performance qui est choisi par l'utilisateur.

33. Agencement selon la revendication 32, dans lequel $I_b(\theta_{tr})$ correspond à un niveau de fond dans le système d'agencement de sorte que l'inégalité se réduit à prévoir un angle de seuil qui satisfait l'inégalité consistant en ce que le rapport signal-sur-fond de la mesure de la luminance provenant de la première couche est supérieur à une certaine valeur spécifiée $F_{tr}$.

Figure 1(a)

Figure 1(b)

Figure 2 (a)

Figure 2 (b)

Figure 3 (a)

Figure 3 (b)

Figure 4(a)

Figure 4(b)

environment

Figure 5

environment

Figure 7

Figure 9 (a)

Figure 9(b)

Figure 9(c)

Figure 6(a)

Figure 6(b)

Figure 8(a)

Figure 8(b)

Figure 10(a)

Figure 10(b)

Figure 11

Figure 12(a)

Figure 12(b)

Figure 12(c)

Figure 13(a)

Figure 13(b)

Figure 14(a)

Figure 14(b)

Figure 15(a)

Figure 15 (b)

Figure 15 (c)

Figure 15 (d)

1610    1605

1600

Figure 16

Figure 17

1700

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4810658 A

- US 5192510 A

**Non-patent literature cited in the description**

- **POLERECKY et al.** *Applied Optics,* 01 August 2000, vol. 39 (22), 3968-3977

- **POLERECKY et al.** *Applied Optics,* 2000